(19) Europäisches Patentamt
European Patent Office
Office européen des brevets

(11) Veröffentlichungsnummer: **0 017 132**
**B1**

(12) # EUROPÄISCHE PATENTSCHRIFT

(45) Veröffentlichungstag der Patentschrift: **14.10.81**

(51) Int. Cl.³: **C 09 B 57/04**, C 07 D 209/44, D 06 P 3/52

(21) Anmeldenummer: **80101558.7**

(22) Anmeldetag: **25.03.80**

(54) **Neue Farbstoffe, deren Herstellung und Verwendung.**

(30) Priorität: **29.03.79 DE 2912428**

(43) Veröffentlichungstag der Anmeldung: **15.10.80 Patentblatt 80/21**

(45) Bekanntmachung des Hinweises auf die Patenterteilung: **14.10.81 Patentblatt 81/41**

(84) Benannte Vertragsstaaten: **CH DE FR GB IT**

(56) Entgegenhaltungen:
**DE-A-1 670 748**
**DE-C-1 811 796**

(73) Patentinhaber: **BASF Aktiengesellschaft, Carl-Bosch-Strasse 38, D-6700 Ludwigshafen (DE)**

(72) Erfinder: **Neumann, Peter, Dr., Franz-Schubert-Strasse 1, D-6903 Wiesloch (DE)**
Erfinder: **Elser, Wolfgang, Dr., Theodor-Heuss-Strasse 4, D-6706 Wachenheim (DE)**
Erfinder: **Bock, Gustav, Dr., Waldstrasse 16, D-6730 Neustadt 11 (DE)**
Erfinder: **Kermer, Wolf-Dieter, Dr., Im Schlittweg 4, D-6701 Fussgoenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Neue Farbstoffe, deren Herstellung und Verwendung

Die Erfindung betrifft neue Farbstoffe auf der Basis von Isoindolenin sowie ein Verfahren zur Herstellung der Farbstoffe und deren Verwendung.

Die neuen Farbstoffe haben die allgemeine Formel

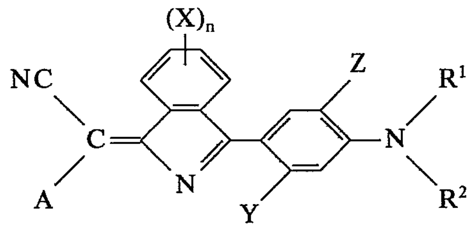

(I)

in der

A Cyan, Carbo-$C_1$- bis $C_4$-Alkoxy, Carbamoyl, N–$C_1$- bis $C_4$-Alkyl-carbamoyl, worin der Alkylrest gegebenenfalls durch $C_1$- bis $C_4$-Alkoxy substituiert ist, N-Phenylcarbamoyl, worin der Phenylrest gegebenenfalls durch $C_1$- bis $C_4$-Alkyl oder $C_1$- bis $C_4$-Alkoxy substituiert ist, Acetyl, Benzoyl, 4-Nitrophenyl oder 4-Cyanphenyl,

X Wasserstoff, Chlor, $C_1$- bis $C_4$-Alkyl, $C_1$- bis $C_4$-Alkoxy, wobei bei $n = 2$ die Substituenten gleich oder verschieden sein können,

n 1 oder 2,

$R^1$ Wasserstoff, Methyl, Äthyl oder 2-Hydroxyäthyl und

$R^2$ Phenyl, durch $C_1$- bis $C_4$-Alkyl oder $C_1$- bis $C_4$-Alkoxy substituiertes Phenyl oder Cyclohexyl oder

$R^1$ Wasserstoff und

$R^2$ $C_1$- bis $C_4$-Alkyl oder

$R^1$ und $R^2$ $C_1$- bis $C_6$-Alkyl; durch Chlor, Cyan, Hydroxy, $C_1$- bis $C_4$-Alkoxy, Phenoxy, $C_2$- bis $C_5$-Alkanoyloxy, das gegebenenfalls durch $C_1$- bis $C_4$-Alkoxy oder Phenoxy substituiert ist oder durch Carbo-$C_1$- bis $C_4$-Alkoxy substituiertes $C_1$- bis $C_4$-Alkyl; Allyl oder Phenyl-$C_1$- bis $C_4$-Alkyl oder die Gruppe

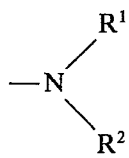

einen gesättigten heterocyclischen Fünf- oder Sechsring, die noch ein Sauerstoff- oder ein weiteres Stickstoffatom als Ringglied enthalten können,

Y Wasserstoff, Hydroxy, Methyl, Äthyl;

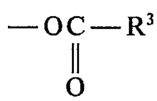

worin $R^3$ für lineares oder verzweigtes $C_1$- bis $C_{12}$-Alkyl, Phenyl oder durch $C_1$- bis $C_4$-Alkyl oder $C_1$- bis $C_4$-Alkoxy substituiertes Phenyl steht;

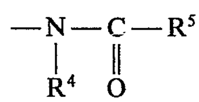

worin $R^4$ für Wasserstoff oder $C_1$- bis $C_4$-Alkyl und $R^5$ für Wasserstoff, lineares oder verzweigtes $C_1$- bis $C_{12}$-Alkyl, Trifluormethyl, Chlormethyl, $C_1$- bis $C_4$-Alkoxymethyl, Phenoxymethyl, wobei im Phenoxy gegebenenfalls 1 oder 2 Wasserstoffatome durch Chlor, Methoxy, Nitro oder $C_1$- bis $C_4$-Alkyl substituiert sind und bei zwei Substituenten diese gleich oder verschieden sein können; Phenylthiomethyl, worin das Phenyl gegebenenfalls durch $C_1$- bis $C_4$-Alkyl substituiert ist; Benzyl; Phenyläthyl; $C_3$- bis $C_7$-Cycloalkyl; Phenyl, durch $C_1$- bis $C_{12}$-Alkyl, $C_1$- bis $C_{12}$-Alkoxy oder Nitro substituiertes Phenyl; $H_5C_6-CH=CH-$; $-CH_2-PO(OR^6)_2$, worin $R^6$ für $C_1$- bis $C_4$-Alkyl steht oder

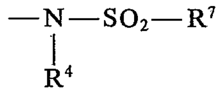

worin $R^4$ die vorstehend genannte Bedeutung hat und $R^7$ für $C_1$- bis $C_{12}$-Alkyl, Phenyl oder $C_1$- bis $C_{12}$-Alkylphenyl steht, oder

Y N – $C_1$- bis $C_4$-Alkylamino, wenn $R^1$ und $R^2$ $C_1$- bis $C_4$-Alkyl sind oder

Y N,N-Di-$C_1$- bis $C_4$-Alkylamino, N-Pyrrolidinyl, N-Piperidinyl oder N-Morpholinyl, wenn

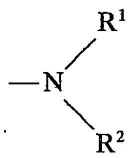

die gleiche Bedeutung hat, und

Z Wasserstoff oder für den Fall, daß $R^1$ und $R^2$ $C_1$- bis $C_4$-Alkyl, Allyl und Y

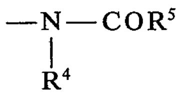

sind, Methoxy oder Äthoxy bedeuten.

Die Farbstoffe färben synthetische Materialien, insbesondere Fasermaterial aus linearen Polyestern aus wäßrigem Bad in blauen bis violetten Farbtönen. Im Falle Z = Methoxy und Äthoxy erhält man Färbungen in Türkistönen. Die Farbstoffe liefern sehr farbstarke Färbungen und erreichen oder übertreffen in dieser Eigenschaft Farbstoffe des Standes der Technik. Die Farbstoffe liefern klare und brillante Farbtöne und weisen gute Thermofixierechtheiten auf. Einige der neuen Farbstoffe eignen sich auch zum Färben von Thermoplasten in der Masse, andere für das in der DE-C-18 11 796 beschriebene Färbeverfahren. Auch hier erhält man brillante Farbtöne.

Als Substituenten kommen für A außer Acetyl, Benzoyl, 4-Nitrophenyl, 4-Cyanphenyl, Carbamoyl und Cyan in Betracht:

a) Carbo-$C_1$- bis $C_4$-Alkoxy wie Carbomethoxy, Carboäthoxy, Carbo-n-propoxy, Carboisopropoxy, Carbo-n-butoxy und Carboisobutoxy;
b) gegebenenfalls im Alkyl substituiertes N-Alkylcarbamoyl wie N-Methyl-, N-Äthyl-, N-Propyl-, N-Butyl-, N-(3-Methoxypropyl)-, N-(3-Äthoxypropyl)-, N-(3-Propoxypropyl)- und N-(3-Butoxypropyl)-carbamoyl;
c) gegebenenfalls im Phenyl substituiertes N-Phenylcarbamoyl wie N-Phenyl, N-(4-Methylphenyl)-, N-(2-Methylphenyl)-, N-(4-Äthylphenyl)-, N-(4-Isopropylphenyl)-, N-(4-Isobutylphenyl)-, N-(4-tert.-Butylphenyl)-, N-(4-Methoxyphenyl)-, N-(4-Äthoxyphenyl)- und N-(4-Butoxyphenyl)-carbamoyl.

Von den für A genannten Substituenten ist Cyan bevorzugt.

Für X sind als Substituenten z. B. im einzelnen zu nennen: Chlor, Methyl, Äthyl, Methoxy, Äthoxy, Butoxy, wobei n = 1 oder 2 ist. Vorzugsweise ist X Wasserstoff.

Für $R^1$ kommen als Substituenten neben Wasserstoff Methyl, Äthyl und 2-Hydroxyäthyl in Betracht, wenn $R^2$ Cyclohexyl, Phenyl oder substituiertes Phenyl wie 4-Methylphenyl, 4-Äthylphenyl, 4-Methoxyphenyl, 4-Äthoxyphenyl, 4-Butoxyphenyl, 4-Isopropylphenyl ist. Wenn $R^1$ Wasserstoff ist, kann $R^2$ auch $C_1$- bis $C_4$-Alkyl, wie Methyl, Äthyl, Propyl, i-Propyl, Butyl, i-Butyl oder tert. Butyl sein.

Für $R^1$ und $R^2$ sind außerdem als Substituenten z. B. im einzelnen zu nennen:

d) gegebenenfalls substituiertes $C_1$- bis $C_6$-Alkyl wie Methyl, Äthyl, n-Propyl, n-Butyl, Pentyl, Hexyl, 2-Chloräthyl, 2-Cyanäthyl, 2-Hydroxyäthyl, 2-Methoxyäthyl, 2-Äthoxyäthyl, 2-Propoxyäthyl, 2-Butoxyäthyl, 2-Phenoxyäthyl, 2-(Äthanoyloxy)-äthyl, 2-(Propanoyloxy)-äthyl, 2-(Butanoyloxy)-äthyl, 2-(Pentanoyloxy)-äthyl; 2-(Methoxyäthanoyloxy)-äthyl, 2-(Äthoxyäthanoyloxy)-äthyl, 2-(Phenoxyäthanoyloxy)-äthyl, 2-(Carbomethoxy)-äthyl, 2-(Carboäthoxy)-äthyl, 2-(Carbobutoxy)-äthyl und 2-(Carbopropoxy)-äthyl;
e) Allyl und Phenyl-$C_1$- bis $C_4$-Alkyl wie Benzyl, $\beta$-Phenyl-äthyl, $\beta$-Phenylpropyl, $\alpha$-Phenylpropyl und Phenylbutyl.
f) Als Vertreter der gesättigten 5- und 6gliedrigen Heterocyclen für

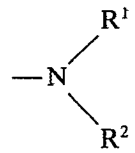

sind z. B. zu nennen: N-Pyrrolidinyl, N-Piperidinyl, N-Morpholinyl, N-Piperazinyl und N'-$C_1$- bis $C_4$-Alkylpiperazinyl mit Methyl, Äthyl, Propyl oder Butyl als Alkylrest am N'-Atom.

Als Substituenten kommen für Y im einzelnen neben den bereits bestimmt genannten z. B. in Betracht:

g)

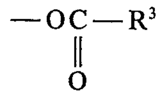

mit $R^3$ = Methyl, Äthyl, Propyl, Butyl, Hexyl, 1-Äthylpentyl, Heptyl, Nonyl, Undecyl, Dodecyl, Phenyl, 4-Methylphenyl, 4-Äthylphenyl, 4-Methoxyphenyl und 4-Äthoxyphenyl;

h)

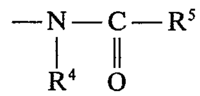

mit $R^4$ = $C_1$- bis $C_4$-Alkyl wie Methyl, Äthyl, Propyl oder vorzugsweise Wasserstoff und $R^5$ = Wasserstoff oder

α) $C_1$- bis $C_{12}$-Alkyl wie Methyl, Äthyl, n-Propyl, Isopropyl, N-Butyl, Isobutyl, 2-Methylpropyl, tert.-Butyl, n-Pentyl, 2,2-Dimethylpropyl, 1-Methylbutyl, 1-Äthylpropyl, 1,1-Dimethylpropyl, n-Hexyl, 1,1-Dimethylbutyl, 1,3-Dimethylbutyl, Heptyl, 1-Äthyl-pentyl, 1-Propyl-butyl, 2,4-Dimethylpentyl, Octyl, Nonyl, Decyl, Undecyl, 1,1-Dimethylnonyl, Dodecyl;

β) Alkoxy-, Phenoxy- und Phenylthiomethyl wie Methoxymethyl, Äthoxymethyl, Propoxymethyl, Butoxymethyl; Phenoxymethyl, 2-, 3- und 4-Methylphenoxymethyl, 2-, 3- und 4-Äthylphenoxymethyl, 4-tert.-Butylphenoxymethyl, 4-Isobutylphenoxymethyl, 4-tert.-Butyl-2-methylphenoxymethyl, 2,3-Dimethylphenoxymethyl, 2,4-Dimethylphenoxymethyl, 2,5-Dimethylphenoxymethyl, 3,5-Dimethylphenoxymethyl, 3,4-Dimethylphenoxymethyl, 2-, 3- und 4-Chlorphenoxymethyl, 2-, 3- und 4-Methoxyphenoxymethyl, 4-Äthoxyphenoxymethyl, 2-Methyl-4-chlorphenoxymethyl, 4-Nitrophenoxymethyl; Phenylthiomethyl, 4-Methylphenylthiomethyl, 4-tert.-Butylphenylthiomethyl;

γ) $C_3$- bis $C_7$-Cycloalkyl wie Cyclopropyl, Cyclobutyl, Cyclopentyl, Cyclohexyl und Cycloheptyl;

δ) Phenyl, durch Alkyl, Alkoxy oder Nitro substituiertes Phenyl wie 2-, 3- und 4-Methylphenyl, 2-, 3- und 4-Äthylphenyl, 2-, 3- und 4-n- oder i-Propylphenyl, 2-, 3- und 4-n- oder i-Butylphenyl, 4-Dodecylphenyl, 4-Methoxyphenyl, 4-Äthoxyphenyl, 4-n- oder i-Propoxyphenyl und 4-n- oder i-Butoxyphenyl;

ε) einen Rest der Formel $-CH_2-PO(OR^6)_2$, mit $R^6$ = Methyl, Äthyl, n- oder i-Propyl und n- oder i-Butyl;

neben den bereits für $R^5$ genannten Gruppen Trifluormethyl, Chlormethyl, Benzyl, Phenyläthyl und $C_6H_5-CH=CH-$.

i)

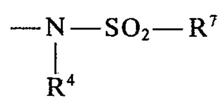

mit $R^4$ = $C_1$- bis $C_4$-Alkyl wie Methyl, Äthyl und Propyl oder vorzugsweise Wasserstoff und $R^7$

α) $C_1$- bis $C_{12}$-Alkyl wie Methyl, Äthyl, Propyl, Butyl, Pentyl, Hexyl, Octyl, Nonyl, Decyl und Dodecyl oder

β) Phenyl, durch $C_1$- bis $C_{12}$-Alkyl substituiertes Phenyl wie 2-, 3- und 4-Methylphenyl, 2-, 3- und 4-Äthylphenyl, 2-, 3- und 4-Isopropylphenyl, 2-, 3- und 4-Isobutylphenyl, 4-Pentylphenyl, 4-Hexylphenyl, 4-Octylphenyl, 4-Nonylphenyl, 4-Decylphenyl und 4-Dodecylphenyl.

Für Y kommt ferner $C_1$- bis $C_4$-Alkylamino wie Methylamino, Äthylamino, Propylamino und Butylamino in Betracht, wenn $R^1$ und $R^2$ $C_1$- bis $C_4$-Alkyl sind.

Außerdem kann Y N,N-Di-$C_1$- bis $C_4$-Alkylamino wie Dimethylamino, Diäthylamino, Dipropylamino und Dibutylamino oder N-Pyrrolidinyl, N-Piperidinyl, N-Morpholinyl oder N'$-C_1$- bis $C_4$-Alkyl-piperazinyl sein, wenn

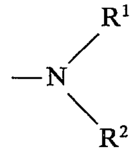

die gleiche Bedeutung hat.

Z steht vorzugsweise für Wasserstoff.

Z kann auch für Methoxy oder Äthoxy stehen, wenn $R^1$ und $R^2$ $C_1$- bis $C_4$-Alkyl oder Allyl und Y eine Gruppe der Formel

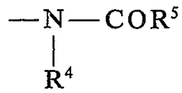

ist.

Von den Verbindungen der Formel I sind solche der Formel Ib)

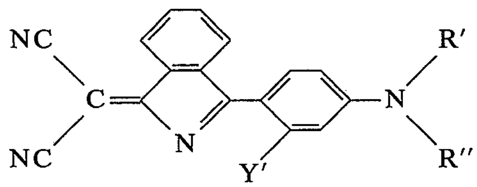

(Ib)

aus wirtschaftlichen und coloristischen Gründen bevorzugt.

In der Formel Ib) steht

Y' für Wasserstoff, Hydroxy,

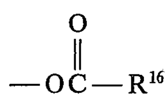

Methyl, $-NH-COR^8$ oder $-NH-SO_2R^9$, worin $R^8$ lineares oder verzweigtes $C_1$- bis $C_{12}$-Alkyl, Methoxymethyl, gegebenenfalls im Phenoxy durch Methoxy oder $C_1$- bis $C_4$-Alkyl substituiertes Phenoxymethyl, gegebenenfalls im Phenyl durch $C_1$- bis $C_4$-Alkyl substituiertes Phenylthiomethyl, Benzyl, Phenyläthyl, Phenyl, $C_1$- bis $C_{12}$-Alkylphenyl, $C_6H_5-CH=CH-$, $C_3$- bis $C_7$-Cycloalkyl, $C_1$- bis $C_4$-Alkoxyphenyl, $-CH_2-PO=(OCH_3)_2$, $-CH_2-PO(OC_2H_5)_2$, $-CH_2-PO(OC_3H_7)_2$ oder $-CH_2-PO=(OC_4H_9)_2$; $R^9$ $C_1$- bis $C_{12}$-Alkyl, Phenyl oder durch $C_1$- bis $C_{12}$-Alkyl substituiertes Phenyl und $R^{16}$ $C_1$- bis $C_6$-Alkyl oder Phenyl bedeuten und

R' und R'' für $C_1$- bis $C_4$-Alkyl, 2-Hydroxyäthyl, $C_1$- bis $C_4$-Alkoxyäthyl, 2-Phenoxyäthyl, 2-Chloräthyl, 2-Cyanäthyl, 2-(Carbomethoxy)-äthyl, 2-(Carboäthoxy)-äthyl, 2-(Propanoyloxy)-äthyl, 2-(Äthanoyloxy)-äthyl, Allyl, Benzyl, wobei die Substituenten R' und R'' gleich oder verschieden sein können oder

R' für Wasserstoff oder Methyl und

R'' für Phenyl, durch Methyl, Äthyl, Methoxy oder Äthoxy substituiertes Phenyl oder Cyclohexyl oder

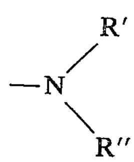

für N-Pyrrolidinyl, N-Piperidinyl oder N-Morpholinyl.

Besonders bevorzugt sind Verbindungen der Formel Ib), in der die Gruppe

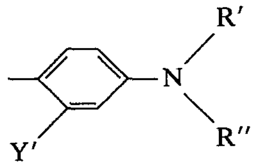

für

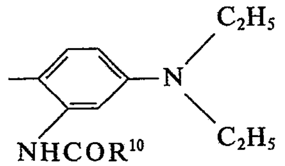

(IIIa)

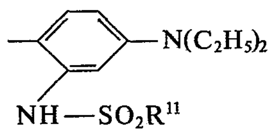

(IIIb)

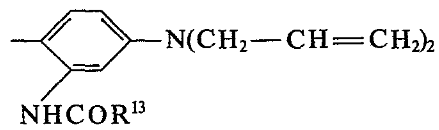

(IIIc)

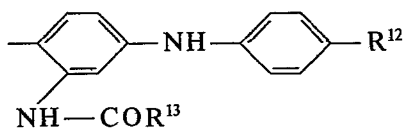

oder

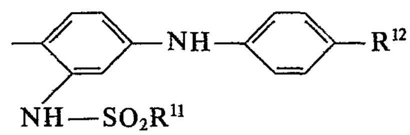

(IIId) (IIIe)

steht, worin

$R^{10}$ lineares oder verzweigtes $C_1$- bis $C_{12}$-Alkyl, Methoxymethyl, Phenoxymethyl, das im Phenoxy gegebenenfalls durch Methoxy oder $C_1$- bis $C_4$-Alkyl substituiert ist, $C_3$- bis $C_7$-Cycloalkyl, Phenyl, durch $C_1$- bis $C_4$-Alkoxy oder $C_1$- bis $C_4$-Alkyl substituiertes Phenyl, Benzyl, Phenyläthyl oder $C_6H_5-CH=CH-$,

$R^{11}$ $C_1$- bis $C_{12}$-Alkyl, Phenyl oder $C_1$- bis $C_{12}$-Alkylphenyl,

$R^{12}$ Wasserstoff, Methyl, Äthyl, Methoxy oder Äthoxy und

$R^{13}$ $C_1$- bis $C_6$-Alkyl bedeuten.

Die Farbstoffe der Formel (I) oder (Ib) können für sich allein oder in Form von Mischungen verschiedener Farbstoffe der Formeln (I) oder (Ib) oder auch in Mischung mit anderen Farbstoffen angewendet werden, z. B. im Falle der Dispersionsfarbstoffe mit anderen Dispersionsfarbstoffen.

Von diesen Farbstoffen sind solche der Formel Ib), in der

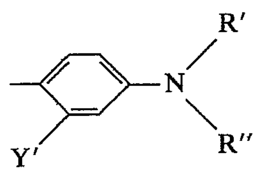

für einen Rest der Formel

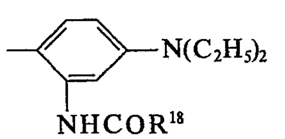

oder

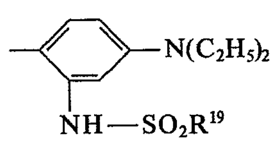

(IIIf) (IIIg)

steht und worin $R^{18}$ $C_1$- bis $C_{12}$-Alkyl oder Phenoxymethyl und $R^{19}$ Methyl oder Äthyl bedeuten, oder Gemische davon, als Farbstoffe zum Färben von Polyesterfasermaterial ganz besonders bevorzugt. Diese Farbstoffe geben besonders farbstarke Färbungen.

Von den zuletzt genannten Farbstoffen sind wiederum solche mit dem Rest der Formel (IIIf) worin $R^{18}$ Methyl, Äthyl bedeutet, oder Gemische dieser Farbstoffe bevorzugt, da diese extrem hohe Farbstärke auf Polyester aufweisen.

Weiterhin sind von den Farbstoffen der Formel Ib) solche, bei denen $R'=R''=$Äthyl und Y'

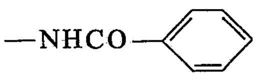
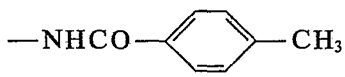

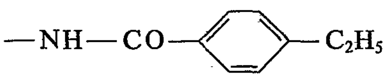
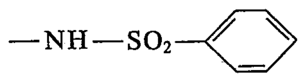

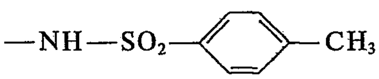
oder
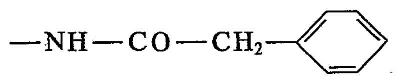

bedeuten, ganz besonders bevorzugt.

Diese Farbstoffe färben Polystyrol in hoher Farbstärke in der Masse.

Die neuen Farbstoffe der Formel (I) werden durch Umsetzen von Isoindolinderivaten der Formel

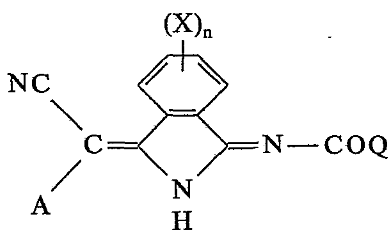
(II)

wobei die Isoindolinderivate auch in einer der tautomeren Formen vorliegen können, mit einem aromatischen Amin der Formel

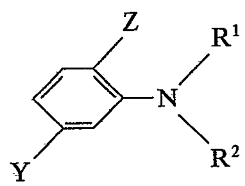
(III)

in einer organischen Flüssigkeit in Gegenwart einer katalytischen Menge einer Säure erhalten. In den Formeln (II) und (III) haben A, X, n, Y, Z, $R^1$ und $R^2$ die oben angegebenen Bedeutungen und Q steht für lineares oder verzweigtes $C_1$- bis $C_{12}$-Alkyl oder für gegebenenfalls durch Methyl, Nitro oder Chlor substituiertes Phenyl.

Die als Ausgangsstoffe benötigten Verbindungen der Formel (II) werden aus 1,3-Diiminoisoindolinen oder deren tautomeren Formen auf folgendem Wege hergestellt:

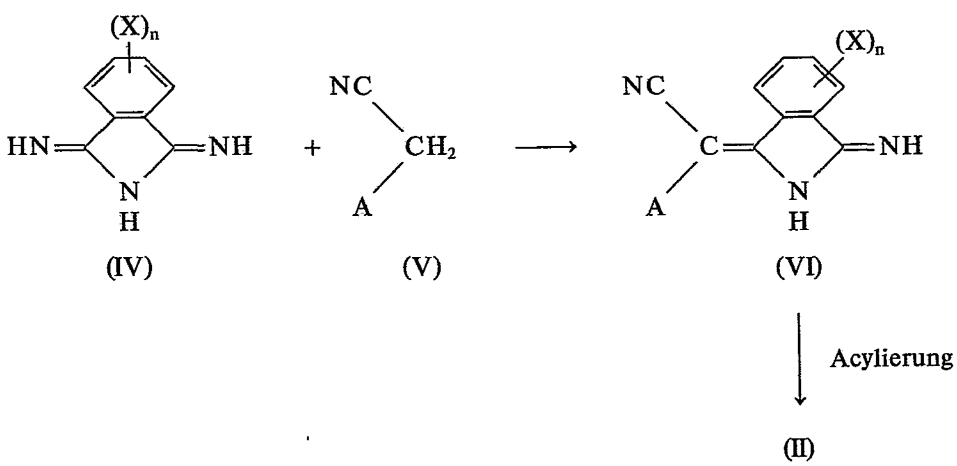

(IV) (V) (VI)

(II)

Die Kondensation von IV oder dessen tautomeren Formen mit der methylenaktiven Verbindung (V) erfolgt in bekannter Weise. Solche Halbumsetzungen von (IV) mit (V) sind z. B. in der DE-A-16 70 748 beschrieben.

Die Acylierung des Halbumsetzungsproduktes (VI) erfolgt in an sich bekannter Weise mit den entsprechenden Carbonsäureanhydriden oder Carbonsäurechloriden.

Die Umsetzung von (VI) mit Carbonsäureanhydriden erfolgt zweckmäßigerweise in einem Überschuß des Reagenz bei Temperaturen zwischen 80 und 150, vorzugsweise zwischen 110 und 130°C. Die Acylierung mit Carbonsäurechloriden, wie Benzoylchlorid erfolgt vorteilhafterweise in inerten organischen Lösungsmitteln, z. B. o-Dichlorbenzol, Chlorbenzol, Nitrobenzol oder Naphthalin, in Gegenwart säurebindender Mittel, wie tertiärer Amine.

Als Acylierungsmittel kommen Carbonsäurechloride oder -anhydride von $C_2$- bis $C_{13}$-Alkansäuren, der Chloressigsäure, Benzoesäure, Methylbenzoesäure, Chlorbenzoesäure oder Nitrobenzoesäure in Betracht.

Als Acylierungsmittel sind aus wirtschaftlichen Gründen solche bevorzugt, die sich von der Essig-, der Propion-, der Butter- und Benzoesäure ableiten.

Als Diimino-isoindoline der Formel (IV), die auch in Form der tautomeren Amino-iminoisoindolenine vorliegen können, kommen z. B. in Betracht: 1,3-Diiminoisoindolin, 1,3-Diimino-5-methylisoindolin, 1,3-Diimino-5-methoxyisoindolin, 1,3-Diimino-5-äthoxyisoindolin, 1,3-Diimino-5-butoxyisoindolin, 1,3-Diimino-5-chlorisoindolin und 1,3-Diimino-4,5-dichlor-indolin.

Als methylenaktive Verbindungen der Formel (V) sind z. B. zu nennen: Malonsäuredinitril, Cyanessigsäure-$C_1$- bis $C_4$-alkylester, wie die Methyl-, Äthyl-, n-Propyl-, Isopropyl-, n-Butyl- oder i-Butylester; Cyanacetamid, Cyanessigsäure-N-$C_1$- bis $C_4$-alkylamide und Cyanessigsäure-N-($C_1$- bis $C_4$-alkoxy-$C_1$- bis $C_4$-alkyl)-amide wie N-Methyl-, N-Äthyl-, N-Propyl-, N-Butyl, N-(3-Methoxypropyl)-, N-(3-Äthoxypropyl)-, N-(3-(Butoxypropyl)-cyanacetamid; Cyanessigsäure-N-phenylamide, die im Phenyl gegebenenfalls durch $C_1$- bis $C_4$-Alkoxy oder $C_1$- bis $C_4$-Alkyl substituiert sind wie N-Phenyl-, N-(4-Methylphenyl)-, N-(4-Äthylphenyl)-N-(4-Methoxyphenyl)- oder N-(4-Äthoxyphenyl)-cyanacetamid; Cyanaceton, ω-Cyanacetophenon; 4-Nitrophenyl-acetonitril, 4-Cyanphenyl-acetonitril.

Als Ausgangskomponenten der Formel (III) kommen solche in Betracht, die der entsprechenden Gruppe in der Formel (I) entsprechen und worin Y, Z, $R^1$ und $R^2$ die für diese Substituenten angegebenen Bedeutungen haben können.

Als aromatische Amine sind solche der Formel

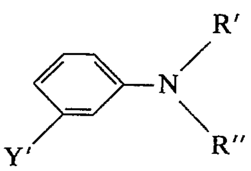

(IIIa)

bevorzugt.

In der Formel IIIa stehen Y' für Wasserstoff, Hydroxy, Methyl oder für eine der Gruppen der Formeln

$$-NH-CO-R^8,\ -NH-SO_2-R^9 \text{ oder } -O\overset{O}{C}-R^{16}$$

worin $R^8$ lineares oder verzweigtes $C_1$- bis $C_{12}$-Alkyl, Methoxymethyl, gegebenenfalls im Phenoxy durch Methoxy oder $C_1$- bis $C_4$-Alkyl substituiertes Phenoxymethyl, gegebenenfalls im Phenyl durch $C_1$- bis $C_4$-Alkyl substituiertes Phenylthiomethyl, Benzyl, Phenyläthyl, Phenyl, $C_1$- bis $C_{12}$-Alkylphenyl, $C_6H_5-CH=CH$; $C_3$- bis $C_7$-Cycloalkyl, $C_1$- bis $C_4$-Alkoxyphenyl, $-CH_2PO(OCH_3)_2$, $-CH_2PO(OC_2H_5)_2$, $-CH_2PO(OC_3H_7)_2$ oder $-CH_2PO(OC_4H_9)_2$, $R^9$ $C_1$- bis $C_{12}$-Alkyl, Phenyl oder durch $C_1$- bis $C_{12}$-Alkyl substituiertes Phenyl, $R^{16}$ $C_1$- bis $C_6$-Alkyl oder Phenyl bedeuten und R' und R'' für $C_1$- bis $C_4$-Alkyl, 2-Hydroxyäthyl, $C_1$- bis $C_4$-Alkoxyäthyl, 2-Phenoxyäthyl, 2-Chloräthyl, 2-Cyanäthyl, 2-(Carbomethoxy)äthyl, 2-(Carboäthoxy)-äthyl, 2-(Propanoyloxy)-äthyl, 2-(Äthanoyloxy)-äthyl, Allyl oder Benzyl, wobei die Substituenten R' und R'' gleich oder verschieden sein können, oder R' auch für Wasserstoff oder Methyl, wenn R'' Phenyl, durch Methyl, Äthyl, Methoxy oder Äthoxy substituiertes Phenyl oder Cyclohexyl ist oder

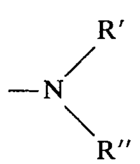

für N-Pyrrolidinyl, N-Piperidinyl oder N-Morpholinyl stehen.

Zur Herstellung der Farbstoffe (I) wird das Isoindolinderivat der Formel (II) mit dem aromatischen Amin (III), das in mindestens der stöchiometrisch erforderlichen Menge angewendet wird, bei Temperaturen zwischen 90 und 150, vorzugsweise zwischen 110 und 130°C umgesetzt (kondensiert).

Die Kondensation erfolgt in organischen Lösungsmitteln, wie Acetanhydrid, Propionsäureanhydrid, Buttersäure-, Isobuttersäure-, Benzoesäureanhydrid, aromatischen Kohlenwasserstoffen, z. B. Benzol,

Toluol, Xylol, Mesitylen, Chlorbenzol, Dichlorbenzol, Trichlorbenzol, Nitrobenzol, Naphthalin, Tetrahydrothiophen-S-dioxid, Dimethylformamid, Dimethylacetamid, N-Methylpyrrolidon oder Gemischen davon.

Beim Arbeiten in Carbonsäureanhydriden können an der Komponente der Formel (III) Acylierung oder Umacylierung eintreten, die sich bei entsprechender Reaktionsführung z. B. zur Darstellung von Farbstoffgemischen nutzen lassen.

Als katalytisch wirkende Säuren sind vor allem Mineralsäure, aromatische und aliphatische Sulfonsäuren und stark saure Carbonsäuren, wie konzentrierte Schwefelsäure, Phosphorsäure, Chlorwasserstoff, Chloressigsäure, Dichloressigsäure, Trichloressigsäure, Trifluoressigsäure, Trifluormethansulfonsäure, Benzolsulfonsäure, Toluolsulfonsäure, Methansulfonsäure, bevorzugt.

Bei der Synthese von (I) ist es nicht erforderlich, die acylierten Monokondensationsprodukte (II) zu isolieren. Man kann mit gleichem Erfolg auch das Halbkondensationsprodukt (VI) zusammen mit dem Amin (III) in einem Acylierungsmittel, z. B. Acetanhydrid oder Propionsäureanhydrid in Gegenwart katalytischer Mengen einer Säure direkt zu (I) umsetzen.

Man kann auch so verfahren, daß zunächst (VI) mit dem Carbonsäureanhydrid umgesetzt und danach dem Reaktionsgemisch das Amin (III) und die katalytische Menge an Säure zugegeben wird. Durch Erhitzen erfolgt dann die Umsetzung zum Farbstoff (I).

Die Isolierung der Farbstoffe erfolgt in üblicher Weise durch Filtrieren des Reaktionsgemisches und Waschen des Filtergutes mit leicht entfernbaren, den Farbstoff nicht lösenden organischen Flüssigkeiten, wie Methanol, Äthanol, Isopropanol.

Die Erfindung soll durch die folgenden Ausführungsbeispiele weiter erläutert werden. Die im folgenden angegebenen Teile und Prozentangaben beziehen sich auf das Gewicht. Die Raumteile verhalten sich zu den Teilen wie das Liter zum Kilogramm.

In den Beispielen wurden die folgenden Verbindungen mit römischen Ziffern bezeichnet:

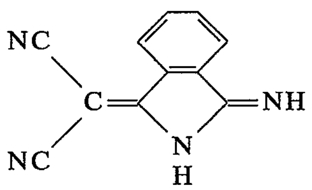

(VII)

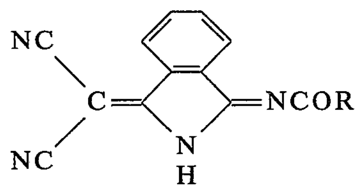

(VIII)

VIII a) R = $-CH_3$

b) R = $-C_2H_5$

c) R = 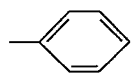

d) R = 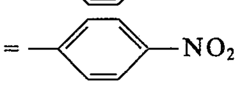

Beispiel 1

50 Teile Verbindung VII werden in 250 Raumteilen Essigsäureanhydrid 45 Minuten bei 120° C gerührt. Man erhält nach Absaugen, Waschen mit Methanol und Trocknen 55 Teile der Verbindung VIII a) vom Schmelzpunkt 226–228° C.

Beispiel 2

87 Teile Verbindung VII werden in 250 Raumteilen Propionsäureanhydrid 30 Minuten auf 120–130° C erhitzt. Nach Abkühlen wird abgesaugt, mit Methanol gewaschen und getrocknet. Man erhält 94,6 Teile Verbindung VIIIb) vom Schmelzpunkt 208–210° C.

Beispiel 3

Eine Mischung aus 87 Teilen Verbindung VII und 300 Teilen Benzoesäureanhydrid wird eine Stunde bei 120–125° C gerührt. Danach versetzt man bei etwa 80° C mit 500 Raumteilen Methanol, läßt abkühlen, saugt den Niederschlag ab und trocknet nach dem Waschen mit Methanol. Man isoliert 103 Teile der Verbindung VIIIc) vom Schmelzpunkt 226–228° C.

Beispiel 4

Eine Mischung aus 9,7 Teilen Verbindung VII und 150 Raumteilen Toluol sowie 50 Raumteilen trockenem Pyridin wird mit 9,68 Teilen Benzoylchlorid versetzt und 1,5 Stunden zum Sieden erhitzt. Vom Ungelösten wird heiß abfiltriert, der aus dem Filtrat auskristallisierende Niederschlag abgesaugt, mit Methanol gewaschen und getrocknet. Ausbeute an Verbindung VIIIc): 6,9 Teile vom Schmelzpunkt 225–227° C.

Beispiel 5

Eine Mischung aus 9,7 Teilen Verbindung VII, 100 Raumteilen Toluol, 50 Raumteilen Dimethylacetamid, 50 Raumteilen trockenem Pyridin wird mit 11,3 Teilen 4-Nitrobenzoylchlorid versetzt und 3,5 Stunden unter Rückfluß erhitzt. Man fügt nochmals 11,3 Teile des Säurechlorids zu und hält 2 Stunden bei Siedetemperatur. Man versetzt vorsichtig mit 200 Raumteilen Methanol, saugt nach einstündigem Rühren ab, wäscht mit Methanol und trocknet. Man erhält 11,7 Teile Verbindung VIIId) vom Schmelzpunkt 254 – 256° C.

Beispiel 6

9,7 Teile Verbindung VII und 6,2 Teile N,N-Dimethylanilin werden in 150 Raumteilen Essigsäureanhydrid in Gegenwart einiger Tropfen konzentrierter Schwefelsäure 1,5 Stunden auf 120° C erhitzt. Nach Abkühlen wird abgesaugt, mit Essigsäureanhydrid und Methanol gewaschen und getrocknet. Man erhält 7,7 Teile Farbstoff der Formel Ib) mit Y' = H und R' = R'' = $CH_3$, der Polyester in rotstichig blauen Tönen färbt.

Beispiel 7

9,65 Teile Verbindung VII werden in 100 Raumteilen Propionsäureanhydrid 30 Minuten auf 120° C erhitzt. Darauf fügt man 7,97 Teile N,N-Diäthylanilin und zwei Tropfen konzentrierte Schwefelsäure zu und rührt 3 Stunden bei 120° C. Nach Abkühlen wird abgesaugt, mit Methanol und etwa 1000 Raumteilen warmem Wasser gewaschen und getrocknet. Ausbeute: 7,3 Teile Farbstoff der Formel Ib) mit Y' = H und R' = R'' = $C_2H_5$, der Polyester in rotstichig blauen Tönen färbt.

Beispiel 8

a) 9,7 Teile Verbindung VII werden mit 12,5 Teilen 3-Diäthylaminoacetanilid in 70 Raumteilen Essigsäureanhydrid und einigen Tropfen konzentrierter Schwefelsäure 2,5 Stunden auf 120 – 130° C erhitzt. Man engt unter vermindertem Druck ein, saugt den nach Abkühlen erhaltenen Niederschlag ab und wäscht ihn mehrmals mit Essigsäureanhydrid und wenig Methanol. Nach Trocknen erhält man 4,1 Teile Farbstoff der Formel Ib) mit Y' = $NHCOCH_3$ und R' = R'' = $C_2H_5$, der Polyester in blauen Tönen färbt.

b) 6,3 Teile Verbindung VIIIb), 6,3 Teile 3-Diäthylaminoacetanilid, 40 Raumteile o-Dichlorbenzol und zwei Tropfen konzentrierte Schwefelsäure werden 45 Minuten bei 120° C gerührt. Nach dem Absaugen, Waschen mit Methanol und Trocknen erhält man 1,6 Teile Farbstoff der Formel Ib) mit Y' = $NHCOCH_3$ und R' = R'' = $C_2H_5$.

c) 7,45 Teile Verbindung VIIIc) und 6,3 Teile 3-Di-äthylaminoacetanilid (83%ig) werden in 20 Raumteilen Tetrahydrothiophendioxid und drei Tropfen konzentrierter Schwefelsäure auf 120 – 130° C erwärmt, bis im Dünnschichtchromatogramm das Ausgangsmaterial verschwunden ist. Nach Isolierung analog Beispiel 8b) erhält man 3,33 Teile Farbstoff der Formel Ib) mit Y' = $NHCOCH_3$ und R' = R'' = $C_2H_5$.

Beispiel 9

a) 5 Teile Verbindung VIIIb) und 4,8 Teile 3-Diäthylaminopropionsäureanilid werden in 20 Raumteilen Toluol und zwei Tropfen konzentrierter Schwefelsäure 2 Stunden unter Rückfluß erhitzt. Nach Abkühlen, Absaugen, Waschen mit Methanol und warmem Wasser und Trocknen erhält man 4,9 Teile Farbstoff der Formel Ib) mit Y' = $NHCOC_2H_5$ und R' = R'' = $C_2H_5$, der Polyester in blauen Tönen färbt.

b) 7,5 Teile Verbindung VIIIb), 6,7 Teile 3-Diäthylaminopropionsäureanilid werden in 30 Raumteilen Mesitylen und zwei Tropfen konzentrierter Schwefelsäure 2,5 Stunden auf 120° C erhitzt. Nach Aufarbeitung analog Beispiel 9a) isoliert man 6,9 Teile Farbstoff der Formel Ib) mit Y' = $NHCOC_2H_5$ und R' = R'' = $C_2H_5$.

c) Zu 5,8 Teilen 3-Diäthylaminoanilin tropft man langsam 20 Raumteile Propionsäureanhydrid. Nach einstündigem Nachrühren bei 50° C gibt man 6,25 Teile Verbindung VIIIb) und eine Spatelspitze Chloressigsäure zu. Man hält 30 Minuten bei 120 – 130° C und arbeitet nach Abkühlen analog Beispiel 9a) auf. Man erhält 6,8 Teile Farbstoff Ib) mit Y' = $NHCOC_2H_5$ und R' = R'' = $C_2H_5$.
Verfährt man wie oben beschrieben, verwendet aber an Stelle der Chloressigsäure einige Tropfen Trifluoressigsäure, so erhält man 6,7 Teile, mit o-Phosphorsäure 8 Teile des gleichen Farbstoffs.

d) Eine Mischung aus 3,43 Teilen der Verbindung VIIId), 2,64 Teilen 3-Diäthylaminopropionsäureanilid, 10 Raumteilen Xylol und 10 Raumteilen Dimethylacetamid und 1 Tropfen konzentrierter

Schwefelsäure hält man 1,5 Stunden bei 125°C. Die Aufarbeitung erfolgt analog Beispiel 9a). Ausbeute: 0,9 Teile Farbstoff der Formel Ib) $Y' = NHCOC_2H_5$ und $R' = R'' = C_2H_5$.

Beispiel 10

Zu 8,2 Teilen 3-Diäthylaminoanilin tropft man langsam 25 Raumteile Propionsäureanhydrid, rührt 1 Stunde bei 50°C nach und setzt darauf der Reaktionslösung 12,5 Teile 3-Diäthylaminoacetanilid, 19,2 Teile Verbindung VII sowie einige Tropfen konzentrierte Schwefelsäure zu. Das Gemisch wird 1,5 Stunden bei 130°C gerührt. Die nach dem Abkühlen ausgefallenen Kristalle werden isoliert, mit Essigsäureanhydrid, Methanol und warmem Wasser gewaschen und getrocknet. Man erhält 8,2 Teile Farbstoff. Der Farbstoff ist ein Gemisch aus 2 Komponenten der Formel Ib) mit $R' = R'' = C_2H_5$ und $Y' = NHCOCH_3$ und $Y' = NHCOC_2H_5$. Das Farbstoffgemisch färbt Polyester in blauen Tönen.

Beispiel 11

10 Teile 3-Diäthylaminoanilin werden in 25 Raumteilen Ameisensäure 45 Minuten unter Rückfluß erhitzt. Der nach Einengen verbleibende Rückstand wird mit 9,5 Teilen Verbindung VII, 40 Raumteilen Propionsäureanhydrid und drei Tropfen konzentrierter Schwefelsäure versetzt. Nach zweistündigem Rühren bei 120°C läßt man das Gemisch erkalten, saugt ab und wäscht den Niederschlag mit Äthanol. Das Filtergut wird anschließend mit Wasser ausgerührt, abgesaugt, mit Wasser gewaschen und getrocknet. Man erhält 2,7 Teile Farbstoff. Der Farbstoff ist ein Gemisch aus 2 Komponenten der Formel Ib) mit $Y' = -NH-CHO$ und $-NH-COC_2H_5$, $R' = R'' = C_2H_5$. Das Gemisch färbt Polyester in blauen Tönen an.

Beispiel 12

Eine Mischung aus 13,3 Teilen Verbindung VII, 18,7 Teilen 3'-Diäthylaminobuttersäureanilid, 70 Raumteilen Propionsäureanhydrid und einigen Tropfen konzentrierter Schwefelsäure wird 30 Minuten auf 120°C erwärmt. Nach dem Erkalten saugt man ab, wäscht den Rückstand mit Methanol und warmem Wasser und trocknet. Man erhält 14,4 Teile des Farbstoffs der Formel Ib) mit $Y' = -NHCOCH_2CH_2CH_3$ und $R' = R'' = -C_2H_5$, der Polyester in blauen Tönen färbt.

Beispiel 13

Verwendet man 20,9 Teile 3'-Diäthylaminocapronsäureanilid und arbeitet im übrigen analog Beispiel 12, so erhält man 18,7 Teile eines Farbstoffgemisches, das Polyester in blauen Farbtönen färbt. Das Gemisch enthält 2 Farbstoffe der Formel Ib) mit $R' = R'' = -C_2H_5$ und $Y' = -NHCO(CH_2)_4CH_3$ und $Y' = -NHCOC_2H_5$.

Beispiel 14

Verwendet man 22 Teile 3'-Diäthylaminooenanthsäureanilid und arbeitet im übrigen analog Beispiel 12, so erhält man 15,6 Teile eines Gemisches aus Farbstoffen der Formel Ib) mit $-NHCO(CH_2)_5CH_3$ und $-NHCOC_2H_5$ und $R' = R'' = C_2H_5$, das Polyester in blauen Tönen färbt.

Beispiel 15

Verwendet man 23,2 Teile 3'-Diäthylaminocaprylsäureanilid und arbeitet im übrigen analog Beispiel 12, so erhält man 18,1 Teile eines Gemiches aus zwei Farbstoffen der Formel Ib) mit $Y' = -NHCO(CH_2)_6CH_3$ und $-NHCOC_2H_5$ und $R' = R'' - C_2H_5$, der Polyester in blauen Tönen färbt.

Beispiel 16

Eine Mischung aus 11,4 Teilen Verbindung VII, 14,9 Teilen 3'-Diäthylaminovaleriansäureanilid, 30 Raumteilen Propionsäureanhydrid und zwei Tropfen konzentrierter Schwefelsäure wird in ein auf 120°C erwärmtes Ölbad getaucht. Nach 15 Minuten läßt man erkalten und arbeitet nach Beispiel 12 auf. Man erhält 12,2 Teile des Farbstoffs der Formel Ib) mit $Y' = -NHCO(CH_2)_3CH_3$ und $R' = R'' - C_2H_5$, der Polyester in blauen Tönen färbt.

### Beispiel 17

Verwendet man 15,2 Teile 3'-Diäthylaminpelargonsäureanilid und 9,5 Teile Verbindung VII und arbeitet im übrigen analog Beispiel 16, so erhält man 11,2 Teile des Farbstoffs der Formel Ib) mit $Y = -NHCO(CH_2)_7CH_3$ und $R' = R'' - C_2H_5$, der Polyester in blauen Tönen färbt.

### Beispiel 18

Verwendet man 15,9 Teile 3'-Diäthylaminocaprinsäureanilid und 9,5 Teile Verbindung VII und arbeitet im übrigen analog Beispiel 16, so erhält man 11,1 Teile des Farbstoffs der Formel Ib) mit $Y' = -NHCO(CH_2)_8CH_3$ und $R' = R'' = C_2H_5$, der Polyester in blauen Tönen färbt.

### Beispiel 19

Verwendet man 17,1 Teile 3'-Diäthylaminolaurinsäureanilid und 9,5 Teile Verbindung VII und arbeitet im übrigen analog Beispiel 16, so erhält man 9,8 Teile des Farbstoffs der Formel Ib) mit $Y' = -NHCO(CH_2)_{10}CH_3$ und $R' = R'' = -C_2H_5$, der Polyester in blauen Tönen färbt.

Die nach den Beispielen 16 bis 19 dargestellten Farbstoffe enthalten nur sehr geringe Mengen des Farbstoffs der Formel Ib) mit $Y' = -NHCOC_2H_5$ und $R' = R'' = -C_2H_5$.

### Beispiel 20

Verwendet man 11,8 Teile 3'-Diäthylaminomethoxyessigsäureanilid und 9,5 Teile Verbindung VII und arbeitet im übrigen analog Beispiel 16, so erhält man 8 Teile des Farbstoffgemisches, das Farbstoffe der Formel Ib) mit $Y' = -NHCOCH_2OCH_3$ und $-NHCOC_2H_5$ und $R' = R'' - C_2H_5$. Das Gemisch färbt Polyester in blauen Tönen an.

### Beispiel 21

9,7 Teile Verbindung VII, 12,5 Teile 3'-Diäthylaminopivalinsäureanilid, 40 Raumteile Propionsäureanhydrid und etwa ein Raumteil konzentrierte Schwefelsäure werden 1,5 Stunden auf 120 bis 130°C erhitzt. Die Lösung wird unter vermindertem Druck teilweise eingeengt, der erhaltene Niederschlag abgesaugt und mit Essigsäureanhydrid, Methanol und Wasser gewaschen. Den Rückstand digeriert man mit etwa 20 Teilen Essigsäureanhydrid, saugt ab und wäscht mit Methanol und warmem Wasser. Man erhält 6,55 Teile des Farbstoffs der Formel Ib) mit $Y' = -NHCOC(CH_3)_3$ und $R' = R'' - C_2H_5$, der Polyester in blauen Tönen färbt.

### Beispiel 22

7,76 Teile Verbindung VII und 17,7 Teile 3'-Diäthylamino-2-äthylhexansäureanilid werden in 25 Raumteilen Propionsäureanhydrid mit einigen Tropfen konzentrierter Schwefelsäure 1,5 Stunden auf 130°C erhitzt. Das Gemisch wird unter vermindertem Druck eingeengt und in Wasser gegossen. Nach längerem Stehen saugt man die Fällung ab. Das Nutschgut wird mit Methanol, Essigsäure, warmem Wasser gewaschen und getrocknet. Man erhält 2,6 Teile des Farbstoffes der Formel Ib) mit

$$Y' = NHCO-\underset{\displaystyle C_2H_5}{\underset{|}{CH}}-(CH_2)_3CH_3 \quad \text{und} \quad R' = R'' = C_2H_5$$

der Polyester in blauen Tönen färbt.

### Beispiel 23

Eine Mischung aus 20,9 Teilen 3'-Diäthylamino-3,3-dimethyl-propionsäureanilid, 13,3 Teilen Verbindung VII und 70 Raumteilen Propionsäureanhydrid sowie einigen Tropfen konzentrierter Schwefelsäure werden 30 Minuten auf 120°C erwärmt. Nach Aufarbeitung analog Beispiel 12 erhält man 15,8 Teile Farbstoffgemisch, das zwei Farbstoffe der Formel Ib) mit $Y' = -NHCOCH_2C(CH_3)_3$ und $-NHCOC_2H_5$ und $R' = R'' = -C_2H_5$ enthält. Das Gemisch färbt Polyester in blauen Tönen an.

## Beispiel 24

Aus 9,5 Teilen Verbindung VII und 14,1 Teilen 3'-Diäthylaminophenylessigsäureanilid erhält man beim Arbeiten nach Beispiel 16 9,9 Teile des Farbstoffs der Formel Ib) mit $Y' = -NHCOCH_2C_6H_5$ und $R' = R'' = -C_2H_5$, der Polyester in blauen Tönen färbt.

## Beispiel 25

Aus 9,5 Teilen Verbindung VII und 14,9 Teilen 3'-Diäthylamino-4-methoxybenzoesäureanilid erhält man beim Arbeiten nach Beispiel 16 13,1 Teile des Farbstoffs der Formel Ib) mit $Y' = -NHCO-C_6H_4-4-OCH_3$ und $R' = R'' = -C_2H_5$, der Polyester in blauen Tönen färbt.

## Beispiel 26

Eine Mischung aus 7,8 Teilen 3'-Diäthylaminotrifluoressigsäureanilid, 8,94 Teilen Verbindung VIIIc), 30 Raumteilen Tetrahydrothiophendioxid und zwei Tropfen konzentrierter Schwefelsäure wird 30 Minuten auf 120°C erhitzt. Der nach mehrtägigem Stehen ausgefallene Niederschlag wird abgesaugt, gewaschen und getrocknet. Man erhält 1,2 Teile des Farbstoffs der Formel Ib) mit $Y' = -NHCOCF_3$, $R' = R'' = -C_2H_5$, der Polyester in rotstichigblauen Tönen färbt.

## Beispiel 27

9,7 Teile Verbindung VII und 17,8 Teile 3'-Diäthylaminophenoxyessigsäureanilid werden in 50 Raumteilen Essigsäureanhydrid in Gegenwart einiger Tropfen konzentrierter Schwefelsäure 75 Minuten auf 120 bis 130°C erhitzt. Man saugt nach Erkalten ab, wäscht mit Essigsäureanhydrid und Methanol und trocknet. Man erhält 8 Teile Farbstoff der Formel Ib) mit $Y' = -NHCOCH_2OC_6H_5$ und $R' = R'' = -C_2H_5$, der Polyester in blauen Tönen färbt.

## Beispiel 28

Eine Mischung aus 34,5 Teilen 3'-Diäthylamino-(4-chlor-2-methylphenoxyessigsäure)anilid, 19 Teilen Verbindung VII, 90 Raumteilen Propionsäureanhydrid und einigen Tropfen konzentrierter Schwefelsäure wird 1,5 Stunden auf 120°C erhitzt. Nach Aufarbeitung analog Beispiel 12 erhält man 21,7 Teile des Farbstoffs der Formel Ib) mit

$Y' = -NH-CO-CH_2-O-$ 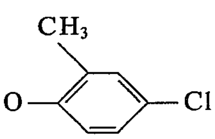 und $R' = R'' = -C_2H_5$

der Polyester in blauen Tönen färbt.

## Beispiel 29

Eine Mischung aus 9 Teilen Verbindung VIIIb), einem Überschuß an rohem 3'-Diäthylamino-phenylthioessigsäure-anilid (erhalten aus 3'-Diäthylaminochloressigsäureanilid und Thiophenol), 25 Raumteilen Xylol und 2 Tropfen konzentrierter Schwefelsäure wird 1 Stunde auf 110–120°C erwärmt. Nach der Zugabe von 20 Raumteilen Dimethylacetamid hält man noch 45 Minuten bei 120°C und kühlt darauf ab. Der gebildete Niederschlag wird abgesaugt, mit Methanol gewaschen, mit etwa 500 Raumteilen Wasser verrührt, abgesaugt und mit warmem Wasser gewaschen und getrocknet. Man erhält 6,24 Teile des Farbstoffs, der Polyester in blauen Tönen färbt. Der Farbstoff besteht im wesentlichen aus der Verbindung der Formel Ib) mit

$Y' = -NH-CO-CH_2-S-$ 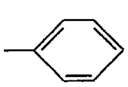 und $R' = R'' = -C_2H_5$

Beispiel 30

Eine Mischung aus 6,25 Teilen Verbindung VIIIb), einem Überschuß an rohem

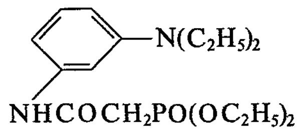

(dargestellt aus 3'-Diäthylaminochloressigsäureanilid und Triäthylphosphit), 20 Raumteilen Xylol und einigen Tropfen konzentrierter Schwefelsäure wird 1,5 Stunden am Rückfluß erhitzt. Nach Abkühlen gießt man das Reaktionsgemisch in verdünnte Sodalösung, saugt nach dem Stehen über Nacht ab, wäscht den Rückstand mit Wasser und rührt ihn nochmals mit heißem Wasser aus. Nach Trocknen erhält man 7,2 Teile Farbstoff, der Polyester in blauen Tönen färbt. Der Farbstoff besteht im wesentlichen aus der Verbindung der Formel Ib) mit $Y' = -NHCO-CH_2-PO(OC_2H_5)_2$ und $R' = R'' = -C_2H_5$.

Beispiel 31

14,5 Teile 3'-Diäthylamino-methansulfonsäureanilid, 10,4 Teile Verbindung VII, 50 Raumteile Propionsäureanhydrid und drei Tropfen konzentrierter Schwefelsäure werden 1,5 Stunden auf 120°C erwärmt. Nach Aufarbeitung analog Beispiel 12 erhält man 9,8 Teile des Farbstoffs der Formel Ib) mit $Y' = -NHSO_2CH_3$ und $R' = R'' = -C_2H_5$, der Polyester in rotstichigblauen Tönen färbt. Der Farbstoff enthält geringe Anteile des Farbstoffs mit $Y' = -NHCOC_2H_5$.

Beispiel 32

Eine Mischung aus 13,3 Teilen Verbindung VII, 50 Raumteilen Propionsäureanhydrid und drei Tropfen konzentrierter Schwefelsäure wird rasch auf 120°C erwärmt. Bei dieser Temperatur gibt man innerhalb von 10 Minuten 20,3 Teile 3'-Diallylaminopropionsäureanilid zu, hält noch 30 Minuten bei 120–130°C und arbeitet auf. Man erhält 14,3 Teile Farbstoff, der Polyester in rotstichigblauen Tönen färbt. Der Farbstoff besteht zum größten Teil aus der Verbindung Ib) mit $Y' = -NH-COC_2H_5$ und $R' = R'' = -CH_2-CH=CH_2$.

Beispiel 33

5 Teile Verbindung VIIIb) und 5,12 Teile 3-N,N-Diäthyl)-amino-acetanilid werden in 20 Raumteilen Essigsäureanhydrid mit 2 Tropfen konzentrierter Schwefelsäure 1 Stunde unter Rückfluß erhitzt. Der nach mehrtägigem Stehen ausgefallene Niederschlag wird nach Beispiel 12 isoliert. Man erhält 0,85 Teile Farbstoff, der Polyester in violetten Tönen färbt. Der Farbstoff besteht aus einem Gemisch der Verbindungen der Formel Ib) mit $Y' = -NH-CO-CH_3$ und R' und $R'' = -C_2H_4-OH$ und $-C_2H_4OCOCH_3$.

Beispiel 34

19,4 Teile Verbindung VII, 23 Teile N-Methyl-4-äthoxydiphenylamin, 100 Raumteile Essigsäureanhydrid und 1 Raumteil konzentrierte Schwefelsäure werden 1 Stunde auf 120°C erhitzt. Nach Abkühlen wird abgesaugt, mit Methanol gewaschen und getrocknet. Man erhält 19 Teile Farbstoff, der Polyester in blauen Tönen färbt. Der Farbstoff besteht im wesentlichen aus der Verbindung der Formel Ib) mit

$Y' = H$, $R' = -CH_3$ und $R'' =$ 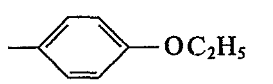

Beispiel 35

Eine Mischung aus 5,76 Teilen Verbindung VIIIc), 3,42 Teilen N,N-Diäthyl-m-toluidin, 20 Raumteilen Propionsäureanhydrid und zwei Tropfen konzentrierter Schwefelsäure wird eine Stunde auf 120°C erhitzt. Nach Zugabe eines weiteren Teils N,N-Diäthyl-m-toluidin hält man noch 2 Stunden bei dieser Temperatur und arbeitet nach dem Abkühlen nach Beispiel 12 auf. Man erhält 3,55 Teile des Farbstoffs der Formel Ib) mit $Y' = -CH_3$ und $R' = R'' = -C_2H_5$, der Polyester in rotstichigblauen Tönen färbt.

Beispiel 36

7,5 Teile Verbindung VIIIc) und 6,1 Teile der Verbindung der Formel

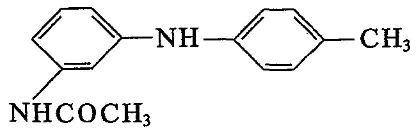

werden in 25 Raumteilen Xylol mit zwei Tropfen konzentrierter Schwefelsäure 2 Stunden auf 120°C erhitzt. Nach Abkühlen wird abgesaugt, mit Dimethylformamid und Methanol gewaschen und getrocknet. Man erhält 3,7 Teile des Farbstoffs der Formel Ib) mit $Y' = -NHCOCH_3$, $R' = H$ und $R'' = -C_6H_4-4-CH_3$, der Polyester in blauen Tönen färbt.

Beispiel 37

13,3 Teile Verbindung VII werden in 40 Raumteilen Essigsäureanhydrid, dem zwei Tropfen konzentrierte Schwefelsäure zugesetzt sind, rasch auf 120°C erhitzt. In die Reaktionsmischung trägt man innerhalb 10 Minuten portionsweise 18,8 Teile 3-Diäthylamino-4-methoxyacetanilid ein. Man rührt noch 2 Stunden bei dieser Temperatur und arbeitet wie üblich auf. Man erhält 9 Teile des Farbstoffs der Formel I mit $A = -CN$, $Z = -OCH_3$, $Y = -NHCOCH_3$ und $R^1 = R^2 = -C_2H_5$, der Polyester in Türkistönen färbt.

Beispiel 38

13,2 Teile 3-Diäthylaminoanilin werden langsam mit 30 Raumteilen Propionsäureanhydrid versetzt; die Lösung wird nach einstündigem Rühren bei 50°C mit 20,8 Teilen der Verbindung

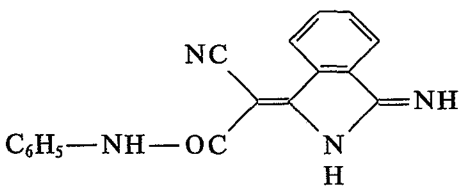

20 Raumteilen Propionsäureanhydrid und drei Tropfen konzentrierter Schwefelsäure versetzt. Nach 4,5 Stunden bei 120°C läßt man erkalten, saugt ab und gießt das Filtrat in Wasser. Der langsam kristallisierende Niederschlag wird abgesaugt und mit warmem Wasser gewaschen; anschließend wird er in 1%iger Natronlauge ausgerührt, abgesaugt, mit Wasser und Methanol gewaschen und getrocknet. Man erhält 31 Teile Farbstoff, der Polyester in violetten Tönen färbt. Der Farbstoff besteht im wesentlichen aus der Verbindung der Formel I mit $A = -CONH-C_6H_5$; $Y = -NHCOC_2H_5$, $Z = H$ und $R^1$ und $R^2 = C_2H_5$.

Beispiel 39

9,9 Teile Verbindung VII, 7 Teile 3-Dimethylaminophenol, 200 Raumteile Essigsäureanhydrid und etwa 1 Raumteil konzentrierte Schwefelsäure werden 3 Stunden auf 100–120°C erhitzt. Anschließend engt man teilweise ein, kühlt das Reaktionsgemisch im Eisbad und saugt den gebildeten Niederschlag ab, wäscht ihn mit wenig Essigsäureanhydrid und Methanol und trocknet. Die Ausbeute an Farbstoff beträgt 7 Teile; er färbt Polyester in violetten Tönen an. Der Farbstoff hat die Formel Ib) mit $Y' = OH$ und $R'$ und $R'' = -CH_3$.

Beispiel 40

9,7 Teile Verbindung VII, 10 Teile 3-Morpholinophenol, 75 Raumteile Essigsäureanhydrid und 0,5 Raumteile konzentrierte Schwefelsäure werden 1,5 Stunden auf 120–130°C erhitzt. Nach dem Abkühlen isoliert man nach dem Aufarbeiten analog Beispiel 39, 5,65 Teile des Farbstoffs der Formel Ib) mit

$Y' = -OH$ und $-OCOCH_3$ und 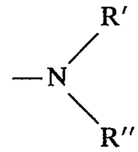 = 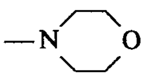

der Polyester in violetten Tönen färbt.

Beispiel 41

4,85 Teile Verbindung VII und 20 Raumteile Propionsäureanhydrid werden 30 Minuten auf 120 C erwärmt. Nach Zugabe von 4,58 Teilen 3-Pyrrolidinophenol, 40 Raumteilen Propionsäureanhydrid und 2 Tropfen konzentrierter Schwefelsäure erwärmt man eine Stunde auf 120° C. Nach Aufarbeitung erhält man 4,46 Teile eines Gemisches von 2 Farbstoffen der Formel Ib) mit

$Y' = OH$ und $-OCOC_2H_5$ und 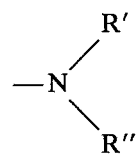 = 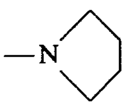

das Polyester in violetten Tönen färbt.

Beispiel 42

6,2 Teile Verbindung VIIIb), 5,44 Teile N-Äthyl-N-(2-chloräthyl)-anilin, 20 Raumteile Propionsäureanhydrid und 2 Tropfen konzentrierte Schwefelsäure werden 15 Minuten auf 120° C erwärmt. Man erhält 5 Teile des Farbstoffs der Formel Ib) mit $Y' = H$ und $R' = -C_2H_5$, $R'' = -CH_2CH_2Cl$, der Polyester in rotstichigblauen Tönen färbt.

Beispiel 43

9,7 Teile Verbindung VII, 14 Teile 3'-Diäthylaminobenzoesäureanilid, 45 Raumteile Propionsäureanhydrid und einige Tropfen konzentrierte Schwefelsäure werden 1,5 Stunden auf 130° C erwärmt. Nach dem Abkühlen saugt man ab, wäscht mit Essigsäure, mit Methanol, warmem Wasser, darauf mit wenig Dimethylformamid und wieder mit Methanol und Wasser und trocknet. Man erhält 8,9 Teile des Farbstoffs der Formel Ib) mit $Y' = -NHCOC_6H_5$ und $R' = R'' = -C_2H_5$, der Polystyrol in blauen Tönen färbt.

Beispiel 44

9,5 Teile Verbindung VII, 14,2 Teile 3'-Diäthylamino-p-tolylsäureanilid, 35 Raumteile Propionsäureanhydrid und einige Tropfen konzentrierte Schwefelsäure werden 1,5 Stunden auf 130° C erwärmt. Nach Absaugen, Waschen mit Methanol und Wasser und Trocknen erhält man 10,1 Teile des Farbstoffs der Formel Ib) mit

$Y' = -NHCO-$ 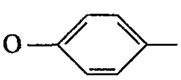 $-CH_3$ und $R' = R'' = -C_2H_5$

der Polystyrol in blauen Tönen färbt.

Beispiel 45

6,2 Teile Verbindung VIIIb) und 7,8 Teile 3'-Diäthylamino-4-äthylbenzoesäureanilid werden in 20 Raumteilen Propionsäureanhydrid und zwei Tropfen konzentrierter Schwefelsäure 30 Minuten auf 130° C erwärmt. Nach der Aufarbeitung entsprechend Beispiel 44 erhält man 6,87 Teile des Farbstoffs der Formel Ib) mit

$Y' = -NHCO-$ 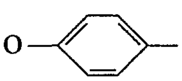 $-C_2H_5$ und $R' = R'' = -C_2H_5$

der Polystyrol in blauen Tönen färbt.

Beispiel 46

Aus 1,93 Teilen Verbindung VIIIb) und 2,4 Teilen 3'-Diäthylamino-4-nitrobenzoesäureanilid erhält man analog Beispiel 45 2 Teile des Farbstoffs der Formel Ib) mit

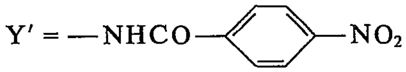 und $R' = R'' = -C_2H_5$

der Polystyrol in blauen Tönen färbt.

Beispiel 47

9,7 Teile Verbindung VII, 15 Teile 3'-Diäthylaminozimtsäureanilid, 38 Raumteile Propionsäureanhydrid und einige Tropfen konzentrierte Schwefelsäure werden 1 Stunde auf 130°C erwärmt. Nach Aufarbeitung erhält man 10,1 Teile des Farbstoffs der Formel Ib) mit $Y' = -NHCO-CH=CH-C_6H_5$ und $R' = R'' = -C_2H_5$, der Polystyrol in blauen Tönen färbt.

Beispiel 48

9,7 Teile Verbindung VII, 16 Teile 3'-Diäthylamino-4-toluol-sulfonsäureanilid, 20 Raumteile Propionsäureanhydrid und einige Tropfen konzentrierte Schwefelsäure werden 1 Stunde auf 130°C erwärmt. Nach Abkühlen setzt man 10 Raumteile Essigsäure zu, kühlt, saugt den Niederschlag ab und wäscht ihn mit Methanol, dann mit wenig Dimethylformamid, anschließend mit Methanol und warmem Wasser. Nach dem Trocknen erhält man 9,75 Teile des Farbstoffs der Formel Ib) mit

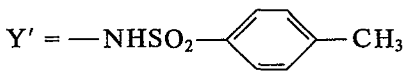 und $R' = R'' = -C_2H_5$

der Polystyrol in rotstichigblauen Tönen färbt.

Beispiel 49

6,25 Teile Verbindung VIIIb), 5,7 Teile N-Äthyl-N-benzyl-anilin, 20 Raumteile Propionsäureanhydrid und 2 Tropfen konzentrierte Schwefelsäure werden 1 Stunde bei 120 bis 130°C gerührt. Nach dem Aufarbeiten erhält man 7,75 Teile des Farbstoffs der Formel Ib) mit $Y' = -H$, $R' = -C_2H_5$ und $R'' = -CH_2C_6H_5$, der Polyester in violetten Tönen färbt.

Beispiel 50

Aus 6,25 Teilen Verbindung VIIIb) und 6,1 Teilen N-Äthyl-N-benzyl-3-methylanilin erhält man analog Beispiel 49 5,1 Teile des Farbstoffs der Formel Ib) mit $Y' = -CH_3$, $R' = -C_2H_5$ und $R'' = -CH_2C_6H_5$, der Polyester in violetten Tönen färbt.

Beispiel 51

5,76 Teile Verbindung VIIIc), 3,9 Teile N,N-Dimethyl-3-methyl-anilin, 20 Raumteile Propionsäureanhydrid und 2 Tropfen konzentrierte Schwefelsäure werden 2 Stunden auf 120 bis 130°C erwärmt. Nach dem Aufarbeiten erhält man 4 Teile des Farbstoffs der Formel Ib) mit $Y' = -CH_3$, $R' = R'' = -CH_3$, der Polyester in violetten Tönen färbt.

Beispiel 52

Aus 5,76 Teilen Verbindung VIIIc) und 4,4 Teilen N,N-Diäthyl-3-methylanilin erhält man analog Beispiel 51 3,55 Teile des Farbstoffs der Formel Ib) mit $Y' = -CH_3$, $R' = R'' = -C_2H_5$, der Polyester in violetten Tönen färbt.

Beispiel 53

Ein Gemisch aus 9,5 Teilen Verbindung VII, 14,1 Teilen 3'-Diäthylaminophenylessigsäureanilid, 30 Raumteilen Propionsäureanhydrid und zwei Tropfen konzentrierter Schwefelsäure wird analog

Beispiel 16 umgesetzt. Man erhält 9,9 Teile des Farbstoffs der Formel Ib) mit $Y' = -NHCOCH_2C_6H_5$ und $R' = R'' = -C_2H_5$, der Polystyrol in blauen Tönen färbt.

Beispiel 54

1,93 Teile Verbindung VIIIc), 2,4 Teile 3'-Diäthylamino-4-nitrobenzoesäureanilid und 1 Tropfen konzentrierte Schwefelsäure werden 2 Stunden auf 120 bis 130°C erwärmt. Man isoliert nach üblicher Aufarbeitung 2 Teile des Farbstoffs der Formel Ib) mit

$Y' =$ 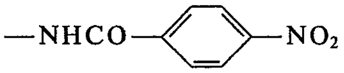 und $R' = R'' = -C_2H_5$

der Polystyrol in blauen Tönen färbt.

Beispiel 55

13,3 Teile Verbindung VII, 50 Raumteile Propionsäureanhydrid und 2 Tropfen konzentrierte Schwefelsäure werden auf 120°C erwärmt. Innerhalb von 5 Minuten fügt man 21,8 Teile 3'-Diäthylaminocyclohexancarbonsäureanilid zu und rührt noch 30 Minuten bei 120°C. Nach üblicher Aufarbeitung erhält man 18,3 Teile des Farbstoffs der Formel Ib) mit

$Y' =$ 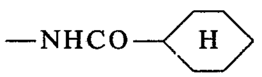 und $R' = R'' = -C_2H_5$

der geringe Anteile des Farbstoffs mit $Y' = -NHCOC_2H_5$ enthält. Die Mischung färbt Polyester in blauen Tönen.

Beispiel 56

2,4 Teile der Verbindung der Formel

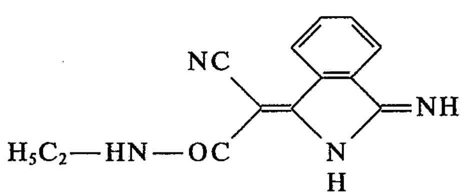

und 15 Raumteile Propionsäureanhydrid werden 30 Minuten auf 110 bis 120°C erwärmt. Nach Zugabe von 3 Teilen 3'-Diäthylamino-phenoxyessigsäureanilid und 2 Tropfen konzentrierter Schwefelsäure rührt man noch 1,5 Stunden bei 120 bis 130°C und saugt nach dem Abkühlen ab. Der Rückstand wird gründlich mit Methanol gewaschen. Die vereinigten Filtrate werden in verdünnte Sodalösung eingerührt. Nach dem Stehen über Nacht saugt man den Niederschlag ab, wäscht mit warmem Wasser und trocknet. Man erhält 2,6 Teile des Farbstoffs der Formel I mit $A = -CONHC_2H_5$, $Y = -NHCOCH_2OC_6H_5$ und $R^1 = R^2 = C_2H_5$, Z = H, der Polyester in violetten Tönen färbt.

**Patentansprüche**

1. Farbstoffe der Formel

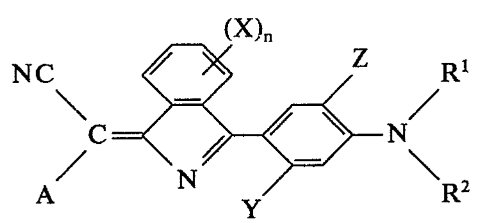

in der

A Cyan, Carbo-$C_1$- bis $C_4$-Alkoxy, Carbamoyl, N-$C_1$- bis $C_4$-Alkylcarbamoyl, worin der Alkylrest gegebenenfalls durch $C_1$- bis $C_4$-Alkoxy substituiert ist, N-Phenylcarbamoyl, worin

der Phenylrest gegebenenfalls durch $C_1$- bis $C_4$-Alkyl oder $C_1$- bis $C_4$-Alkoxy substituiert ist, Acetyl, Benzoyl, 4-Nitrophenyl oder 4-Cyanphenyl,

X Wasserstoff, Chlor, $C_1$- bis $C_4$-Alkyl, $C_1$- bis $C_4$-Alkoxy, wobei bei n=2 die Substituenten gleich oder verschieden sein können,

n 1 oder 2,

$R^1$ Wasserstoff, Methyl, Äthyl oder 2-Hydroxyäthyl und

$R^2$ Phenyl, durch $C_1$- bis $C_4$-Alkyl oder $C_1$- bis $C_4$-Alkoxy substituiertes Phenyl oder Cyclohexyl oder

$R^1$ Wasserstoff und

$R^2$ $C_1$- bis $C_4$-Alkyl oder

$R^1$ und $R^2$ $C_1$- bis $C_6$-Alkyl; durch Chlor, Cyan, Hydroxy, $C_1$- bis $C_4$-Alkoxy, Phenoxy, $C_2$- bis $C_5$-Alkanoyloxy, das gegebenenfalls durch $C_1$- bis $C_4$-Alkoxy oder Phenoxy substituiert ist, oder durch Carbo-$C_1$- bis $C_4$-Alkoxy substituiertes $C_1$- bis $C_4$-Alkyl; Allyl oder Phenyl-$C_1$- bis $C_4$-Alkyl oder die Gruppe

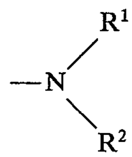

einen gesättigten heterocyclischen Fünf- oder Sechsring, die gegebenenfalls noch ein Sauerstoff- oder ein weiteres Stickstoffatom als Ringglied enthalten,

Y Wasserstoff, Hydroxy, Methyl, Äthyl;

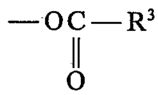

worin $R^3$ für lineares oder verzweigtes $C_1$- bis $C_{12}$-Alkyl, Phenyl oder durch $C_1$- bis $C_4$-Alkyl oder $C_1$- bis $C_4$-Alkoxy substituiertes Phenyl steht;

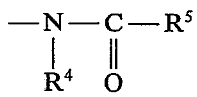

worin $R^4$ für Wasserstoff oder $C_1$- bis $C_4$-Alkyl und $R^5$ für Wasserstoff, lineares oder verzweigtes $C_1$- bis $C_{12}$-Alkyl, Trifluormethyl; Chlormethyl; $C_1$- bis $C_4$-Alkoxymethyl; Phenoxymethyl, bei dem im Phenoxy gegebenenfalls 1 oder 2 Wasserstoffatome durch Chlor, Methoxy, Nitro oder $C_1$- bis $C_4$-Alkyl substituiert sind und die Substituenten gleich oder verschieden sein können; Phenylthiomethyl, worin das Phenyl gegebenenfalls durch $C_1$- bis $C_4$-Alkyl substituiert ist; Benzyl; Phenyläthyl; $C_3$- bis $C_7$-Cycloalkyl; Phenyl, durch $C_1$- bis $C_{12}$-Alkyl oder $C_1$- bis $C_{12}$-Alkoxy oder Nitro substituiertes Phenyl; $H_5C_6-CH=CH-$, oder für $-CH_2-PO(OR^6)_2$ und $R^6$ für $C_1$- bis $C_4$-Alkyl stehen; oder

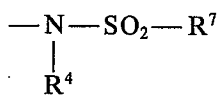

worin $R^4$ die vorstehend genannte Bedeutung hat und $R^7$ für $C_1$- bis $C_{12}$-Alkyl, Phenyl oder $C_1$- bis $C_{12}$-Alkylphenyl steht, oder

Y N-$C_1$- bis $C_4$-Alkylamino, wenn $R^1$ und $R^2$ $C_1$- bis $C_4$-Alkyl sind, oder

Y N,N-Di-$C_1$- bis $C_4$-Alkylamino, N-Pyrrolidinyl, N-Piperidinyl oder N-Morpholinyl, wenn

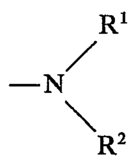

die gleiche Bedeutung hat, und

Z Wasserstoff oder für den Fall, daß $R^1$ und $R^2$ $C_1$- bis $C_4$-Alkyl, Allyl und Y

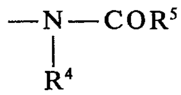

sind, auch Methoxy oder Äthoxy bedeuten.

2. Farbstoffe gemäß Anspruch 1, gekennzeichnet durch die Formel

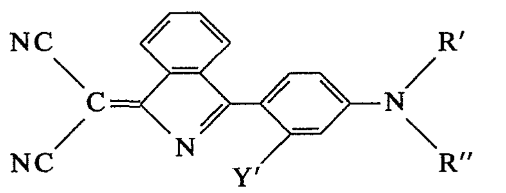

in der

Y' für Wasserstoff, Hydroxy, Methyl,

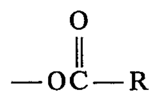 $—NHCOR^8$ oder $—NHSO_2—R^9$

worin

R $C_1$- bis $C_6$-Alkyl oder Phenyl,

$R^8$ lineares oder verzweigtes $C_1$- bis $C_{12}$-Alkyl, Methoxymethyl, gegebenenfalls im Phenoxy durch Methoxy oder $C_1$- bis $C_4$-Alkyl substituiertes Phenoxymethyl, gegebenenfalls im Phenyl durch $C_1$- bis $C_4$-Alkyl substituiertes Phenylthiomethyl, Benzyl, Phenyläthyl, Phenyl, $C_1$- bis $C_{12}$-Alkylphenyl, $C_6H_5—CH=CH—$, $C_3$- bis $C_7$-Cycloalkyl, $C_1$- bis $C_4$-Alkoxyphenyl, $—CH_2—PO(OCH_3)_2$, $—CH_2—PO(OC_2H_5)_2$, $—CH_2—PO(OC_3H_7)_2$ oder $—CH_2—PO(OC_4H_9)_2$ und

$R^9$ $C_1$- bis $C_{12}$-Alkyl, Phenyl oder $C_1$- bis $C_{12}$-Alkylphenyl bedeuten und

R' und R'' für $C_1$- bis $C_4$-Alkyl, 2-Hydroxyäthyl, $C_1$- bis $C_4$-Alkoxyäthyl, 2-Phenoxyäthyl, 2-Chloräthyl, 2-Cyanäthyl, 2-(Carbomethoxy)-äthyl, 2-(Carboäthoxy)-äthyl, 2-(Äthanoyloxy)-äthyl, 2-(Propanoyloxy)-äthyl, Allyl, Benzyl, wobei die Substituenten R' und R'' gleich oder verschieden sein können oder

R' für Wasserstoff oder Methyl und

R'' für Phenyl, durch Methyl, Äthyl, Methoxy oder Äthoxy substituiertes Phenyl oder Cyclohexyl oder

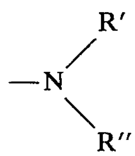

für N-Pyrrolidinyl, N-Piperidinyl oder N-Morpholinyl stehen.

3. Farbstoffe gemäß Anspruch 2, dadurch gekennzeichnet, daß in der Formel der Rest

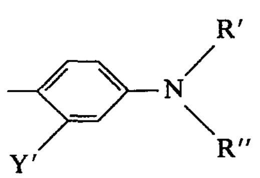

für einen Rest der Formeln

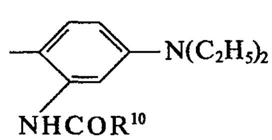

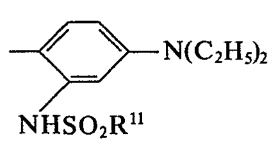

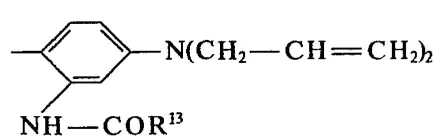

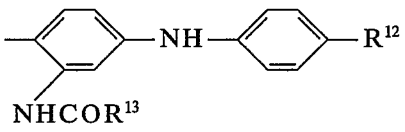 oder 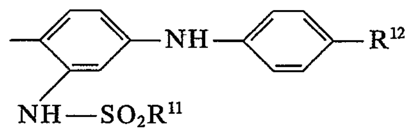

steht, worin

$R^{10}$ lineares oder verzweigtes $C_1$- bis $C_{12}$-Alkyl, Methoxymethyl, Phenoxymethyl, das im Phenoxy gegebenenfalls durch Methoxy oder $C_1$- bis $C_4$-Alkyl substituiert ist, $C_3$- bis $C_7$-Cycloalkyl, Phenyl, durch $C_1$- bis $C_4$-Alkyl oder $C_1$- bis $C_4$-Alkoxy substituiertes Phenyl, Benzyl, Phenyläthyl oder $C_6H_5-CH=CH-$,

$R^{11}$ $C_1$- bis $C_{12}$-Alkyl, Phenyl oder $C_1$- bis $C_{12}$-Alkylphenyl,

$R^{12}$ Wasserstoff, Methyl, Äthyl, Methoxy oder Äthoxy und

$R^{13}$ $C_1$- bis $C_6$-Alkyl bedeuten.

4. Farbstoffe gemäß Anspruch 2, dadurch gekennzeichnet, daß in der angegebenen Formel R′ und R″ Äthyl und Y′ $-NHCOR^{18}$ oder $-NH-SO_2{}^{19}$ bedeuten, worin $R^{18}$ für $C_1$- bis $C_{12}$-Alkyl oder Phenoxymethyl und $R^{19}$ für Methyl oder Äthyl stehen.

5. Farbstoffe gemäß Anspruch 4, dadurch gekennzeichnet, daß R′ und R″ Äthyl und Y′ $-NH-COR^{18}$ bedeuten, worin $R^{18}$ für Methyl oder Äthyl steht.

6. Farbstoffe gemäß Anspruch 2, dadurch gekennzeichnet, daß in der angegebenen Formel R′ und R″ Äthyl und Y′

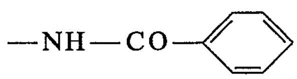 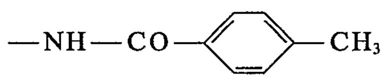

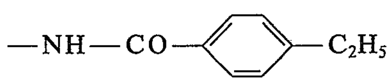 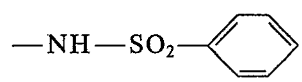

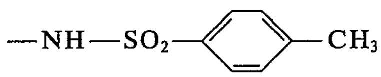 oder 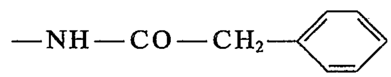

bedeuten.

7. Verfahren zur Herstellung von Farbstoffen gemäß Anspruch 1, dadurch gekennzeichnet, daß man ein Isoindolinderivat der allgemeinen Formel

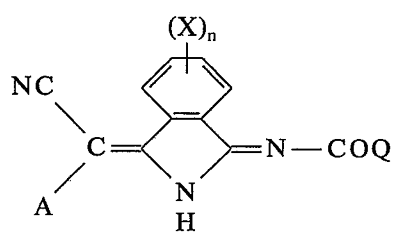

mit einem aromatischen Amin der allgemeinen Formel

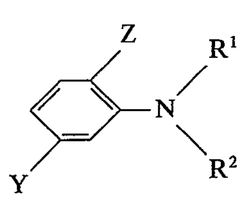

worin A, X, Y, Z, n, $R^1$ und $R^2$ die im Anspruch 1 angegebene Bedeutung haben und Q für lineares oder verzweigtes $C_1$- bis $C_{12}$-Alkyl oder für gegebenenfalls durch Methyl, Nitro oder Chlor substituiertes Phenyl steht, in einer organischen Flüssigkeit in Gegenwart einer katalytischen Menge einer Säure kondensiert.

8. Verwendung der Farbstoffe gemäß den Ansprüchen 1 bis 5 zum Färben von linearen Polyestern oder thermoplastischen Polymeren.

9. Verwendung der Farbstoffe gemäß Anspruch 6 zum Färben von Thermoplasten.

**Claims**

1. A dye of the formula

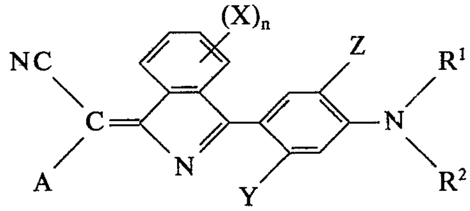

wherein

A is cyano, carbo-$C_1-C_4$-alkoxy, carbamyl, N-$C_1-C_4$-alkyl-carbamyl, where alkyl is unsubstituted or substituted by $C_1-C_4$-alkoxy, N-phenylcarbamyl, where phenyl is unsubstituted or substituted by $C_1-C_4$-alkyl or $C_1-C_4$-alkoxy, acetyl, benzoyl, 4-nitrophenyl or 4-cyanophenyl,

X is hydrogen, chlorine, $C_1-C_4$-alkyl or $C_1-C_4$-alkoxy, and if n is 2 the substituents may be identical of different,

n is 1 or 2,

$R^1$ is hydrogen, methyl, ethyl or 2-hydroxyethyl and and

$R^2$ is phenyl which is unsubstituted or substituted by $C_1-C_4$-alkyl or $C_1-C_4$-alkoxy, or is cyclohexyl, or

$R^1$ is hydrogen and

$R^2$ is $C_1-C_4$-alkyl or

$R^1$ and $R^2$ are $C_1-C_6$-alkyl; $C_1-C_4$-alkyl substituted by chlorine, cyano, hydroxyl, $C_1-C_4$-alkoxy, phenoxy, $C_2-C_5$-alkanoyloxy (which may or may not be substituted by $C_1-C_4$-alkoxy or phenoxy) or carbo-$C_1-C_4$-alkoxy; allyl; or phenyl-$C_1-C_4$-alkvl or

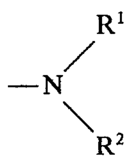

is a saturated heterocyclic five-membered or six-membered ring which may additionally contain an oxygen or a further nitrogen as a ring member,

Y is hydrogen, hydroxyl, methyl or ethyl;

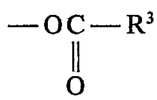

where $R^3$ is linear or branched $C_1-C_{12}$-alkyl or phenyl which is unsubstituted or substituted by $C_1-C_4$-alkyl or $C_1-C_4$-alkoxy;

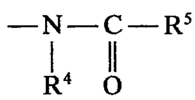

where $R^4$ is hydrogen or $C_1-C_4$-alkyl and $R^5$ is hydrogen, linear or branched $C_1-C_{12}$-alkyl, trifluoromethyl, chloromethyl, $C_1-C_4$-alkoxymethyl or phenoxymethyl (where phenoxy may or may not be substituted by one or two chlorine, methoxy, nitro or $C_1-C_4$-alkyl, and the substituents may be identical or different); phenylthiomethyl, where phenyl is unsubstituted or substituted by $C_1-C_4$-alkyl; benzyl; phenylethyl; $C_3-C_7$-cycloalkyl; phenyl which is unsubstituted or substituted by $C_1-C_{12}$-alkyl, $C_1-C_{12}$-alkoxy or nitro; $H_5C_6-CH=CH-$; $-CH_2-PO(OR^6)_2$, where $R^6$ is $C_1-C_4$-alkyl, or

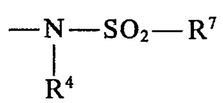

where $R^4$ has the above meanings and

$R^7$ is $C_1-C_{12}$-alkyl, phenyl or $C_1-C_{12}$-alkylphenyl, or

Y is N-$C_1-C_4$-alkylamino when $R^1$ and $R^2$ are $C_1-C_4$-alkyl, or

Y is N,N-di-$C_1-C_4$-alkylamino, N-pyrrolidinyl, N-piperidinyl or N-morpholinyl when

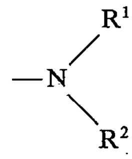

has the same meaning, and

Z is hydrogen or, if $R^1$ and $R^2$ are $C_1-C_4$-alkyl or allyl and Y is

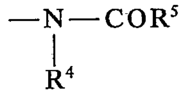

Z is hydrogen, methoxy or ethoxy.

2. A Dye as claimed in claim 1, characterized by the formula

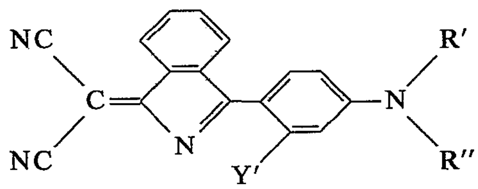

wherein

Y' is hydrogen, hydroxyl, methyl,

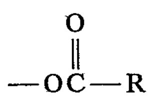 $-NHCOR^8$ or $-NHSO_2-R^9$

where

R is $C_1-C_6$-alkyl or phenyl,

$R^8$ is linear or branched $C_1-C_{12}$-alkyl, methoxymethyl, phenoxymethyl (where phenoxy is unsubstituted or substituted by methoxy or $C_1-C_4$-alkyl), phenylthiomethyl (where phenyl is unsubstituted or substituted by $C_1-C_4$-alkyl), benzyl, phenylethyl, phenyl, $C_1-C_{12}$-alkylphenyl, $C_6H_5-CH=CH-$, $C_3-C_7$-cycloalkyl, $C_1-C_4$-alkoxyphenyl, $-CH_2\text{-}PO(OCH_3)_2$,
$-CH_2-PO(OC_2H_5)_2$, $-CH_2-PO(OC_3H_7)_2$ or $-CH_2-PO(OC_4H_9)_2$ and

$R^9$ is $C_1-C_{12}$-alkyl, phenyl or $C_1-C_{12}$-alkylphenyl and

R' and R'' which may be identical or different, are $C_1-C_4$-alkyl, 2-hydroxyethyl, $C_1-C_4$-alkoxyethyl, 2-phenoxyethyl, 2-chloroethyl, 2-cyanoethyl, 2-(carbomethoxy)-ethyl, 2-(carboethoxy)-ethyl, 2-(ethanoyloxy)-ethyl, 2-(propanoyloxy)-ethyl, allyl or benzyl, or

R' is hydrogen or methyl, and

R'' is phenyl which is unsubstituted or substituted by methyl, ethyl, methoxy or ethoxy, or is cyclohexyl, or

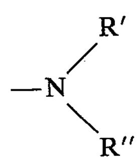

is N-pyrrolidinyl, N-piperidinyl or N-morpholinyl.

3. A dye as claimed in claim 2, characterized in that in the formula the radical

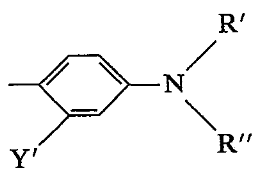

stands for a radical of the formula

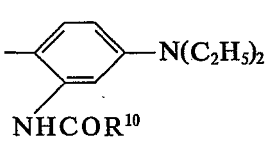 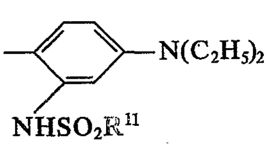 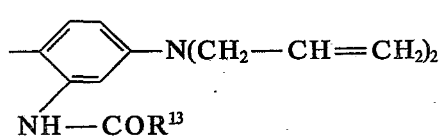

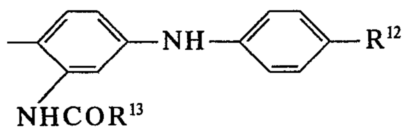

or

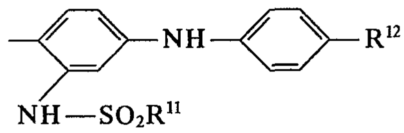

where

| | |
|---|---|
| $R^{10}$ | is linear or branched $C_1-C_{12}$-alkyl, methoxymethyl, phenoxymethyl (where phenoxy is unsubstituted or substituted by methoxy or $C_1-C_4$-alkyl), $C_3-C_7$-cycloalkyl, phenyl (which is unsubstituted or substituted by $C_1-C_4$-alkyl or $C_1-C_4$-alkoxy), benzyl, phenylethyl or $C_6H_5-CH=CH-$, |
| $R^{11}$ | is $C_1-C_{12}$-alkyl, phenyl or $C_1-C_{12}$-alkylphenyl, |
| $R^{12}$ | is hydrogen, methyl, ethyl, methoxy or ethoxy and |
| $R^{13}$ | is $C_1-C_6$-alkyl. |

4. A dye as claimed in claim 2, characterized in that in the specified formula R′ and R″ are ethyl and Y′ is $-NH-COR^{18}$ or $-NH-SO_2R^{19}$, where $R^{18}$ is $C_1-C_{12}$-alkyl or phenoxymethyl and $R^{19}$ is methyl or ethyl.

5. A dye as claimed in claim 4, characterized in that R′ and R″ are ethyl and Y′ is $-NH-COR^{18}$ where $R^{18}$ is methyl or ethyl.

6. A dye as claimed in claim 2, characterized in that in the specified formula R′ and R″ are ethyl and Y′ is

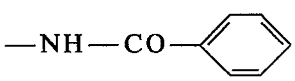

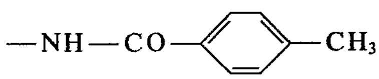

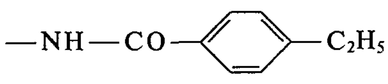

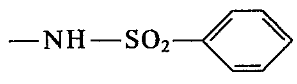

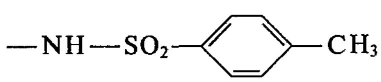

or

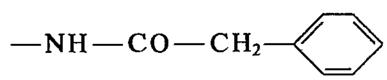

7. A process for the preparation of a dye as claimed in claim 1, characterized in that an isoindoline derivate of the general formula

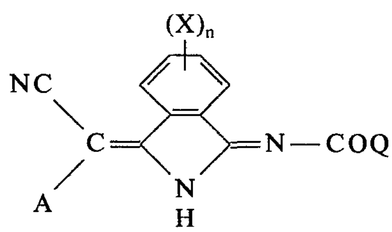

is condensed with an aromatic amine of the general formula

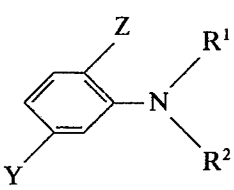

where A, X, Y, Z, n, $R^1$ and $R^2$ have the meanings given in claim 1 and Q is linear or branched $C_1-C_{12}$-alkyl or is phenyl which is unsubstituted or substituted by methyl, nitro or chlorine, in an organic liquid in the presence of a catalytic amount of an acid.

8. Use of a dye as claimed in claims 1 to 5 for dyeing linear polyesters of coloring thermoplastic polymers.

9. Use of a dye as claimed in claim 6 for coloring thermoplastics.

**Revendications**

1. Colorants de formule

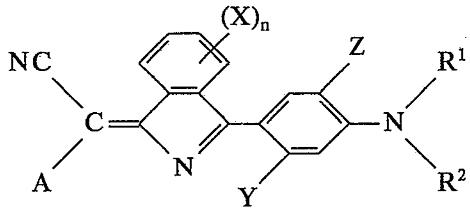

dans laquelle les symboles ont les significations suivantes:

| | |
|---|---|
| A | cyano, carbalcoxy à 1—4 C dans le radical alcoxy, carbamoyle, N-alcoylcarbamoyle à 1—4 C dans l'alcoyle qui est éventuellement substitué par un alcoxy à 1—4 C, N-phénylcarbamoyle, le radical phényle étant éventuellement substitué par un alcoyle à 1—4 C ou un alcoxy à 1—4 C, acétyle, benzoyle, 4-nitrophényle ou 4-cyanophényle; |
| X | hydrogène, chlore, alcoyle à 1—4 C, alcoxy à 1—4 C, les substituants pouvant être semblables ou différents lorsque n=2; |
| n | 1 ou 2; |
| $R^1$ | hydrogène, méthyle, éthyle ou 2-hydroxyéthyle et |
| $R^2$ | phényle, phényle substitué par alcoyle à 1—4 C ou par alcoxy à 1—4 C ou cyclohexyle; ou |
| $R^1$ | hydrogène et |
| $R^2$ | alcoyle à 1—4 C; ou |
| $R^1$ et $R^2$ | alcoyle à 1—6 C; alcoyle à 1—4 C substitué par chlore, cyano, hydroxy, alcoxy à 1—4 C, phénoxy, alcanoyloxy à 2—5 C, qui est éventuellement substitué par un alcoxy à 1—4 C ou un phénoxy, ou carbalcoxy à 1—4 C dans le radical alcoxy; allyle ou phénylalcoyle à 1—4 C dans le radical alcoyle; ou encore le groupe |

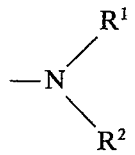

est un noyau pentagonal ou hexagonal hétérocyclique saturé qui peut contenir encore un atome d'oxygène ou un autre atome d'azote en tant que terme cyclique;

Y hydrogène, hydroxy, méthyle, éthyle;

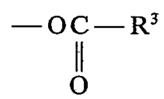

($R^3$ étant un alcoyle à 1—12 C à chaîne linéaire ou ramifiée, un phényle ou un phényle substitué par un alcoyle à 1—4 C ou par un alcoxy à 1—4 C);

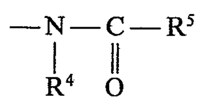

[$R^4$ étant un hydrogène ou un alcoyle à 1—4 C et $R^5$ étant un hydrogène; un alcoyle à 1—12 C à chaîne linéaire ou ramifiée; un trifluorométhyle; un chlorométhyle; un alcoxymethyle à 1—4 C; un phénoxyméthyle, 1 ou 2 atomes d'hydrogène dans le radical phénoxy étant éventuellement substitués par un chlore, un méthoxy, un nitro ou un alcoyle à 1—4 C et les substituants pouvant être semblables ou différents lorsqu'ils sont deux; un phénylthiométhyle, le phényle étant éventuellement substitué par un alcoyle à 1—4 C; un benzyle; un phényléthyle; un cycloalcyle à 3—7 C; un phényle; un phényle substitué par un alcoyle à 1—12 C, par un alcoxy à 1—12 C ou par un nitro; $H_5C_6-CH=CH-$; $-CH-PO(OR^6)_2$ ($R^6$ étant un alcoyle à 1—4 C)]; ou

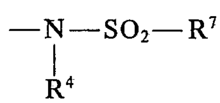

(R4 ayant la signification donné ci-dessus et R7 étant un alcoyle à 1—12 C, un phényle ou un alcoylphényle à 1—12 C dans le radical alcoyle); ou

Y N-alcoylamino à 1—4 C dans le radical alcoyle, lorsque R1 et R2 sont des alcoyles à 1—4 C; ou

Y N,N-dialcoylamino à 1—4 C dans le radical alcoyle, N-pyrrolidinyle, N-pipéridinyle ou N-morpholinyle, lorsque

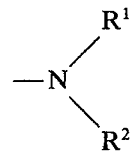

a la même signification; et

Z hydrogène ou, dans le cas où R1 et R2 sont des alcoyles à 1—4 C, des allyles et où

Y = 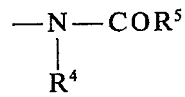

méthoxy ou éthoxy.

2. Colorants selon la revendication 1, caractérisés par la formule

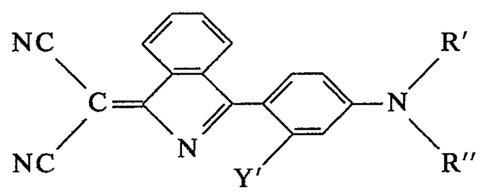

dans laquelle des symboles ont les significations suivantes:

Y' hydrogène, hydroxy, méthyle,

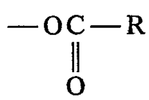 —NHCOR8 ou —$NHSO_2$—R9

(R étant un alcoyle à 1—6 C ou un phényle;

R8 etant un alcoyle à 1—12 C à chaîne linéaire ou ramifiée, un méthoxyméthyle, un phénoxyméthyle éventuallement substitué dans le phénoxy par un méthoxy ou un alcoyle à 1—4 C, un phénylthiométhyle éventuellement substitué dans le phényle par un alcoyle à 1—4 C, un benzyle, un phényléthyle, un phényle, un alcoylphényle à 1—12 C dans le radical alcoyle, $C_6H_5-CH=CH-$, un cycloalcoyle à 3—7 C, un alcoxyphényle à 1—4 C dans le radical alcoxy, $-CH_2-PO(OCH_3)_2$, $-CH_2PO(OC_2H_5)_2$, $-CH_2-PO(OC_3H_7)_2$ ou $-CH_2-PO(OC_4H_9)_2$ et

R9 etant un alcoyle à 1—12 C, un phényle ou un alcoylphényle à 1—12 C dans le radical alcoyle)

R' et R'' alcoyle à 1—4 C, 2-hydroxyéthyle, alcoxyéthyle à 1—4 C, 2-phénoxyéthyle, 2-chloréthyle, 2-cyanéthyle, 2-(carbométhoxy)-éthyle, 2-(carboéthoxy)-éthyle, 2-(éthanoyloxy)-éthyle, 2-(propanoyloxy)-éthyle, allyle, benzyle, les substituants R' et R'' pouvant être semblables ou différents; ou

R' hydrogène ou méthyle et

R'' phényle, phényle substitué par méthyle, éthyle, méthoxy ou éthoxy, ou cyclohexyle; ou encore

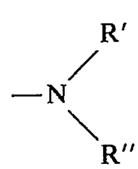

N-pyrrolidinyle, N-pipéridinyle ou N-morpholinyle.

3. Colorants selon la revendication 2, caractérisés en ce que, dans la formule, le radical

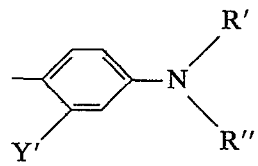

est mis pour un radical de formule

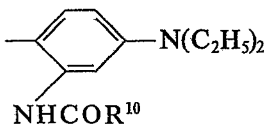 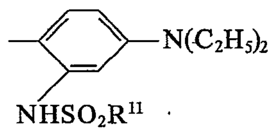 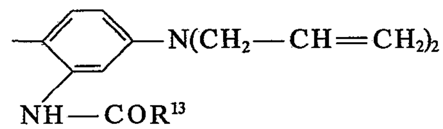

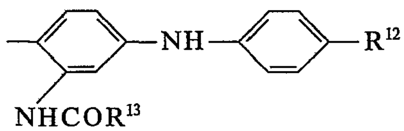 ou 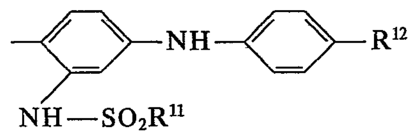

dans laquelle les symboles ont les significations suivantes:

| | |
|---|---|
| $R^{10}$ | alcoyle à 1—12 C à chaîne linéaire ou ramifiée, méthoxyméthyle, phénoxyméthyle qui est éventuellement substitué dans le phénoxy par un méthoxy ou un alcoyle à 1—4 C, cycloalcoyle à 3—7 C, phényle, phényle substitué par alcoxy à 1—4 C ou alcoyle à 1—4 C, benzyle, phényléthyle ou $C_6H_5-CH=CH-$, |
| $R^{11}$ | alcoyle à 1—12 C, phényle ou alcoylphényle à 1—12 C dans le radical alcoyle, |
| $R^{12}$ | hydrogène, méthyle, éthyle, méthoxy ou éthoxy, et |
| $R^{13}$ | alcoyle à 1—6 C. |

4. Colorants selon la revendication 2, caractérisés en ce que, dans la formule indiquée, R′ et R″ représentent chacun un éthyle et Y′ est mis pour $-NHCOR^{18}$ ou $-NH-SO_2R^{19}$, $R^{18}$ étant un alcoyle à 1—12 C ou un phénoxyméthyle et $R^{19}$ étant un méthyle ou un éthyle.

5. Colorants selon la revendication 4, caractérisés en ce que R′ et R″ représentent chacun un éthyle et Y′ un groupe $-NH-COR^{18}$, $R^{18}$ étant mis pour un méthyie ou un éthyle.

6. Colorants selon la revendication 2, caractérisés en ce que, dans la forumel indiquée, R′ et R″ représentent chacun un éthyle et Y′ est mis pour

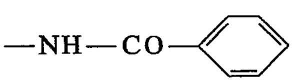 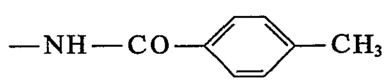

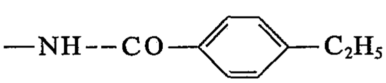 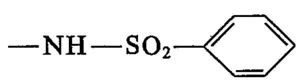

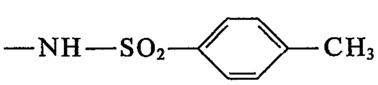 ou 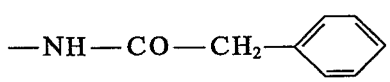

7. Procédé pour la préparation de colorants selon la revendication 1, caractérisé en ce qu'on condense un dérivé de l'isoindoline de formule générale

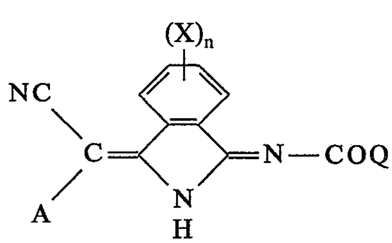

avec une amine aromatique de formule générale

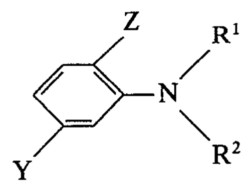

formules dans lesquelles A, X, Y, Z, n, $R^1$ et $R^2$ ont les significations données dans la revendication 1 et Q est mis pour un alcoyle à 1—12 C à chaîne linéaire ou ramifiée ou pour un phényle éventuellement substitué par un méthyle, un nitro ou un chlore, dans un liquide organique et en présence d'une quantité catalytique d'un acide.

8. Utilisation des colorants selon l'une quelconque des revendications 1 à 5 pour la coloration de polyesters linéaires ou de polyères thermoplastiques.

9. Utilisation des colorants selon la revendication 6 pour la coloration de matières thermoplastiques.